# EUROPEAN PATENT APPLICATION

(11) **EP 2 441 835 A1**
(43) Date of publication of application: **18.04.2012**
(21) Application number: 11183734.0
(22) Date of filing: 12.05.2006
(51) Int. Cl.: C12N 15/11, C12Q 1/68, A01N 43/04, C07H 21/04, A61K 31/07, A61K 31/7088, C12N 15/113

(54) **Modulation of stat 6 expression for the treatment of airway hyperresponsiveness**

(30) Priority: 12.05.2005 US 680895 P
(62) Divisional of application: 06759763.3
(71) Applicant: Isis Pharmaceuticals, Inc., Carlsbad, CA 92010 (US)
(72) Inventor: Crosby, Jeffrey, R., San Marcos, CA 92078 (US); Karras, James, G., Murrieta, CA 92562 (US); Freier, Susan, M., Encinitas, CA 92024 (US); Monia, Brett, P., San Diego, CA 92122 (US)
(74) Representative: Carpmaels & Ransford

(57) **Abstract**

Disclosed herein are compounds, compositions and methods for modulating the expression of STAT 6 in a cell, tissue, or animal. Also provided are uses of disclosed compounds and compositions in the manufacture of a medicament for treatment of diseases and disorders related to expression of STAT 6, airway hyperresponsiveness and/or pulmonary inflammation.

## Description

### CROSS-REFERENCE TO RELATED APPLICATIONS

This application claims priority to US Provisional Patent Application Serial Number 60/680,895, filed May 12, 2005 which is incorporated herein by reference in its entirety. This application is related to US Pregrant Publication Nos. 20040115634 and 20050239124, which are hereby incorporated by reference in their entirety.

### INCORPORATION OF SEQUENCE LISTING

A copy of the sequence listing in both a paper and a computer-readable form is provided herewith and hereby incorporated by reference. The computer readable form is provided on 3.5" diskette containing the file named BIOL0062WOSEQ.txt which was created on May 11, 2006.

### BACKGROUND OF THE INVENTION

Allergic rhinitis and asthma are widespread conditions with complex and multifactorial etiologies. The severity of the conditions vary widely between individuals, and within individuals, dependent on factors such as genetics, environmental conditions, and cumulative respiratory pathology associated with duration and severity of disease. Both diseases are a result of immune system hyperresponsiveness to innocuous environmental antigens, with asthma typically including an atopic (i.e., allergic) component.

In asthma, the pathology manifests as inflammation, mucus overproduction, and reversible airway obstruction which may result in scarring and remodeling of the airways. Mild asthma is relatively well controlled with current therapeutic interventions including beta-agonists and low dose inhaled corticosteroids or cromolyn. However, moderate and severe asthma are less well controlled, and require daily treatment with more than one long-term control medication to achieve consistent control of asthma symptoms and normal lung function. With moderate asthma, doses of inhaled corticosteroids are increased relative to those given to mild asthmatics, and/or supplemented with long acting beta-agonists (LABA) (e.g., salmeterol) or leukotriene inhibitors (e.g., montelukast, zafirlukast). Although LABA can decrease dependence on corticosteroids, they are not as effective for total asthma control as corticosteroids (e.g., reduction of episodes, emergency room visits) (Lazarus et al., JAMA. 2001.285: 2583-2593; Lemanske et al., JAMA. 2001. 285: 2594-2603). With severe asthma, doses of inhaled corticosteroids are increased, and supplemented with both LABA and oral corticosteroids. Severe asthmatics often suffer from chronic symptoms, including night time symptoms; limitations on activities; and the need for emergency room visits. Additionally, chronic corticosteroid therapy at any level has a number of unwanted side effects, especially in children (e.g., damage to bones resulting in decreased growth).

Allergic rhinitis is inflammation of the nasal passages, and is typically associated with watery nasal discharge, sneezing, congestion and itching of the nose and eyes. It is frequently caused by exposure to irritants, particularly allergens. Allergic rhinitis affects about 20% of the American population and ranks as one of the most common illnesses in the US. Most suffer from seasonal symptoms due to exposure to allergens, such as pollen, that are produced during the natural plant growth season(s). A smaller proportion of sufferers have chronic allergies due to allergens that are produced throughout the year such as house dust mites or animal dander. A number of over the counter treatments are available for the treatment of allergic rhinitis including oral and nasal antihistamines, and decongestants. Antihistamines are utilized to block itching and sneezing and many of these drugs are associated with side effects such as sedation and performance impairment at high doses. Decongestants frequently cause insomnia, tremor, tachycardia, and hypertension. Nasal formulations, when taken improperly or terminated rapidly, can cause rebound congestion. Anticholinergics and montelukast have substantially fewer side effects, but they also have limited efficacy. Similarly, prescription medications are not free of side effects. Nasal corticosteroids can be used for prophylaxis or suppression of symptoms; however, compliance is variable due to side effects including poor taste and nasal irritation and bleeding. Allergen immunotherapy is expensive and time consuming and carries a low risk of anaphylaxis.

Persistent nasal inflammation can result in the development of nasal polyps. Nasal polyps are present in about 4.2% of patients with chronic rhinitis and asthma (4.4% of men and 3.8% of women) (Grigores et al., Allergy Asthma Proc. 2002, 23:169-174). The presence of polyps is increased with age in both sexes and in patients with cystic fibrosis and aspirin-hypersensitivity triad. Nasal polyposis results from chronic inflammation of the nasal and sinus mucous membranes. Chronic inflammation causes a reactive hyperplasia of the intranasal mucosal membrane, which results in the formation of polyps. The precise mechanism of polyp formation is incompletely understood. Nasal polyps are associated with nasal airway obstruction, postnasal drainage, dull headaches, snoring, anosmia, and rhinorrhea. Medical therapies include treatment for underlying chronic allergic rhinitis using antihistamines and topical nasal steroid sprays. For severe nasal polyposis causing severe nasal obstruction, treatment with short-term steroids may be beneficial. Topical use of cromolyn spray has also been found to be helpful to some patients in reducing the severity and size of the nasal polyps. Oral corticosteroids are the most effective medication for the short-term treatment of nasal polyps, and oral corticosteroids have the best effectiveness in shrinking inflammatory polyps. Intranasal steroid sprays may reduce or retard the growth of small nasal polyps, but they are relatively ineffective in massive nasal polyposis. Although nasal polyps can be treated pharmacologically, many of the therapeutics have undesirable side effects. Moreover, polyps tend to be recurrent, eventually requiring surgical intervention. Compositions and methods to inhibit post-surgical recurrence of nasal polyps are not presently available.

Other diseases characterized by similar inflammatory pathways include, but are not limited to, chronic bronchitis, pulmonary fibrosis, emphysema, chronic obstructive pulmonary disease (COPD), eosinophilic pneumonia, and pediatric asthma.

### Signal Transducer and Activator of Transcription 6 (STAT 6) and inflammatory signaling pathways

It is generally acknowledged that allergy and asthma are a result of the dysregulation of T cell-mediated immunity resulting in a bias towards a Th2 response (enhanced production of interleukin-4 (IL-4) IL-5 and IL-13). The presence of GD4+ T cells producing IL-4, IL-5 and IL-13 cytokines in bronchoalveolar lavage fluid and in airway epithelial biopsies of asthmatics has been clearly documented. STAT 6 is an integral transcription factor involved in interleukin 4 and interleukin 13 signaling. Following activation of their respective receptors, interleukin 4 and interleukin 13 cause their common interleukin 4 receptor alpha chain to become phosphorylated by JAK3 and to subsequently bind to STAT 6. STAT 6 is then phosphorylated by JAK1, homodimerizes and translocates to the nucleus where it binds interleukin 4 response elements and initiates the transcription of a number of genes including IgE (Danahay et al., Inflamm. Res., 2000, 49, 692-699).

STAT 6 (also known as interleukin 4-STAT) was cloned and mapped to chromosome 12q13 (Leek et al., Cytogenet. Cell Genet., 1997, 79, 208-209; Quelle et al., Mol. Cell Biol., 1995, 15, 3336-3343). Nucleic acid sequences encoding STAT 6 are disclosed and claimed in US patent 5,710,266 (McKnight and Hou, 1998).

STAT 6 is primarily expressed as a 4 kb transcript in hematopoietic cells and expressed variably in other tissues (Quelle et al.., Mol. Cell Biol., 1995, 15, 3336-3343). A unique truncated isoform of STAT 6 is expressed in mast cells (Sherman, Immunol. Rev., 2001, 179, 48-56). Disclosed and claimed in PCT publication WO 99/10493 are nucleic acid sequences encoding variants of STAT 6 known as STAT 6b and STAT 6c as well as vectors comprising said nucleic acid sequences (Patel et al., 1999).

STAT 6 knockout mice are viable and develop normally with the exception that interleukin 4 functions are eliminated (Ihle, Curr. Opin. Cell Biol., 2001, 13, 211-217). Additionally, STAT 6 knockout mice fail to develop antigen-induced airway hyper-reactivity in a model of airway inflammation (Kuperman et al., J. Exp. Med.,1998, 187, 939-948).

Inhibition of STAT 6 is expected to attenuate the allergic response and thus, represents an attractive target for drug discovery strategies (Hill et al., Am. J. Respir. Cell Mol. Biol., 1999, 21, 728-737).

Small molecule inhibitors of STAT 6 are disclosed and claimed in PCT publication WO 00/27802 and Japanese Patent JP 2000229959 (Eyermann et al., 2000; Inoue et al., PCT, 2000, Abstract only). Disclosed and claimed in US Patent 6,207,391 are methods for screening modulators of STAT 6 binding to a STAT 6 receptor (Wu and McKinney, 2001).

Wang et al. have demonstrated targeted disruption of STAT 6 DNA-binding activity by a phosphorothioate cis-element decoy oligonucleotide (Wang et al., Blood, 2000, 95, 1249-1257). Hill et al, have used a series of homologous human and murine antisense oligonucleotides targeting STAT 6 to interrupt interleukin 4 and interleukin 13 signaling and attenuate germline C-epsilon transcription in vitro (Hill et al.., Am. J. Respir. Cell Mol. Biol.,1999, 21, 728-737). Subsequently, the in vitro and in vivo pharmacology of three of the antisense oligonucleotides used in the latter study was investigated. Although the oligonucleotides downregulated STAT 6 mRNA, their action was not sufficient to influence alterations in IgE levels in a model of active sensitization (Danahay et al., Inflamm. Res., 2000, 49, 692-699). Although the oligonucleotides were able to decrease target expression in the spleen, splenomegaly was observed, indicating immune-stimulation by the oligonucleotides. US Pregrant Publications Nos. 20040115634 and 20050239124 teach a series of oligonucleotides targeted to STAT 6.

### Antisense oligonucleotides and pulmonary disease

Antisense oligonucleotides (ASOs) are being pursued as therapeutics for pulmonary inflammation, airway hyperresponsiveness, and/or asthma. Lung provides an ideal tissue for aerosolized ASOs for several reasons (Nyce and Metzger, Nature, 1997: 385:721-725, incorporated herein by reference in its entirety); the lung can be targeted non-invasively and specifically, it has a large absorption surface; and is lined with surfactant that may facilitate distribution and uptake of ASOs. Delivery of ASOs to the lung by aerosol results in excellent distribution throughout the lung in both mice and primates. Immunohistochemical staining of inhaled ASOs in normalized and inflamed mouse lung tissue shows heavy staining in alveolar macrophages, eosinophils, and epithelium, moderate staining in blood vessels endothelium, and weak staining in bronchiolar epithelium. ASO- mediated target reduction is observed in dendritic cells, macrophages, eosinophils, and epithelial cells after aerosol administration. The estimated half life of a 2'-methoxyethoxy (2'-MOE) modified oligonucleotide delivered by aerosol administration to mouse or monkey is about 4 to 7, or at least 7 days, respectively. Moreover, ASOs have relatively predictable toxicities and pharmacokinetics based on backbone and nucleotide chemistry. Pulmonary administration of ASOs results in minimal systemic exposure, potentially increasing the safety of such compounds as compared to other classes of drugs.

Compositions and methods for formulation of ASOs and devices for delivery to the lung and nose are well known. ASOs are soluble in aqueous solution and may be delivered using standard nebulizer devices (Nyce, Exp. Opin. Invest. Drugs, 1997, 6:1149-1156). Formulations and methods for modulating the size of droplets using nebulizer devices to target specific portions of the respiratory tract and lungs are well known to those skilled in the art. Oligonucleotides can be delivered using other devices such as dry powder inhalers or metered dose inhalers which can provide improved patient convenience as compared to nebulizer devices, resulting in greater patient compliance.

Generally, the principle behind antisense technology is that an antisense compound hybridizes to a target nucleic acid and effects the modulation of gene expression activity, or function, such as transcription or translation. The modulation of gene expression can be achieved by, for example, target RNA degradation or occupancy-based inhibition. An example of modulation of target RNA function by degradation is RNase H-based degradation of the target RNA upon hybridization with a DNA-like antisense compound. Another example of modulation of gene expression by target degradation is RNA interference (RNAi) using small interfering RNAs (siRNAs). RNAi is a form of antisense-mediated gene silencing involving the introduction of double stranded (ds)RNA-like oligonucleotides leading to the sequence-specific reduction of targeted endogenous mRNA levels. This sequence-specificity makes antisense compounds extremely attractive as tools for target validation and analysis of gene function, as well as therapeutics to selectively modulate the expression of genes involved in diseases.

Antisense oligonucleotides targeted to a number of targets including, but not limited to p38 alpha MAP kinase (US Patent Publication No. 20040171566, incorporated by reference); the CD28 receptor ligands B7.1 and B7.2 (US Patent Publication 20040235164, incorporated by reference); intracellular adhesion molecule (ICAM) (WO 2004/108945, incorporated by reference); and adenosine A₁ receptor (Nyce and Metzger, Nature, 1997, 385:721-725, incorporated herein by reference) have been tested for their ability to inhibit pulmonary inflammation and airway hyperresponsiveness in mouse, rabbit, and/or monkey models of asthma when delivered by inhalation. Various endpoints were analyzed in each case and a portion of the results are presented herein. ASOs targeted to p38 alpha MAP kinase reduced eosinophil recruitment, airway hyperresponsiveness (AHR), and mucus production in two different mouse models. ASOs targeted to each B7.1 and B7.2 decreased target expression and eosinophil recruitment. An ASO targeted to B7.2 also reduced AHR. ASOs targeted to ICAM-1 decreased AHR and decreased neutrophil and eosinophil recruitment in mice. Treatment of Cynomolgus monkeys with an ASO targeted to ICAM-1 significantly reduced airway impedance (resistance) induced by methacholine challenge in naturally Ascaris allergen- sensitized monkeys. An ASO targeted to adenosine A₁ receptor reduced receptor density on airway smooth muscle and reduced AHR in an allergic rabbit model. These data demonstrate that oligonucleotides are effectively delivered by inhalation to cells within the lungs of multiple species, including a non-human primate, and are effective at reducing airway hyperresponsiveness and/or pulmonary inflammation as determined by a number of endpoints.

However, treatment with any ASO targeted to any inflammatory mediator involved in pulmonary inflammation is not always effective at reducing AHR and/or pulmonary inflammation. ASOs targeted to Jun N-terminal Kinase (JNK-1) found to decrease target expression *in vitro* were tested in a mouse model of asthma. Treatment with each of two different antisense oligonucleotides targeted to JNK-1 were not effective at reducing methacholine induced AHR, eosinophil recruitment, or mucus production at any of the ASO doses tested.

A number of ASOs designed to target STAT 6 have been reported for use as research tools. The PCT publication WO0208 8328 (Belardelli et al., 2002) discloses the use of an oligonucleotide of 24 nucleotides in length that is complementary to a nucleic acid molecule encoding STAT 6. US patent 6,699,677 (Schall et al., 2004) discloses the use of an oligonucleotide of 30 nucleotides in length as a PCR primer for amplifying a nucleic acid molecule encoding STAT 6. The PCT publication WO0240647 (Ulrich and Saikh, 2002) discloses the use of an oligonucleotide of 30 nucleotides in length as a PCR primer for amplifying a nucleic acid molecule encoding STAT 6. A series of antisense oliognucleotides targeted to STAT 6 are taught in US Patent Publication US2004-0115634.

The role of STAT 6 in the Th2 inflammatory signaling pathways makes it an attractive therapeutic candidate, as this pathway has been linked to asthma, allergy, and other inflammatory disorders. Currently, there are no known therapeutic agents that effectively inhibit the synthesis of STAT 6, and all investigative strategies to date aimed at modulating function have involved the use of antibodies. Consequently, there remains a need for additional agents capable of effectively inhibiting the activity of STAT 6.

### SUMMARY OF THE INVENTION

The invention provides compounds, particularly antisense compounds, especially nucleic acid and nucleic acid-like oligomers, which are targeted to a nucleic acid encoding STAT 6. Preferably, the antisense compounds are antisense oligonucleotides targeted to STAT 6, particularly human STAT 6 (GenBank Accession No. NM_003153.3, entered October 1, 2002 (SEQ ID NO.1)), that modulate the expression of STAT 6. The compounds comprise at least a 12 nucleobase portion, preferably at least a 15 nucleobase portion, most preferably at least a 17 nucleobase portion targeted to an active target segment, or are at least 80% complementary to at least a 15 nucleobase portion an active target segments.

The invention provides a method for modulating the expression of STAT 6 in cells or tissues comprising contacting the cells with at least one compound of the instant invention, and analyzing the cells for indicators of a decrease in expression of STAT 6 mRNA and/or protein by direct measurement of mRNA and/or protein levels, and/or indicators of pulmonary inflammation and/or airway hyperresponsiveness.

The invention further provides a method for the prevention, amelioration, and/or treatment of pulmonary inflammation and/or airway hyperresponsiveness comprising administering at least one compound of the instant invention to an individual in need of such intervention. The compound is preferably administered by aerosol (i.e., topically) to at least a portion of the respiratory tract. The portion of the respiratory tract selected is dependent upon the location of the inflammation. For example, in the case of asthma, the compound is preferably delivered predominantly to the lung. In the case of allergic rhinitis, the compound is preferably delivered predominantly to the nasal cavity and/or sinus. The compound is delivered using any of a number of standard delivery devices and methods well known to those skilled in the art, including, but not limited to nebulizers, nasal and pulmonary inhalers, dry powder inhalers, and metered dose inhalers.

The invention also provides a method of use of the compositions of the instant invention for the preparation of a medicament for the prevention, amelioration, and/or treatment disease, especially a disease associated with and including at least one indicator of pulmonary inflammation and/or airway hyperresponsiveness. The medicament is preferably formulated for aerosol administration to at least a portion of the respiratory tract.

### DETAILED DESCRIPTION OF THE INVENTION

Asthma, allergy, and a number of other diseases or conditions related to pulmonary inflammation and/or AHR share common inflammatory mediators, including STAT 6, a Th2 cytokine. Therapeutic interventions for these diseases or conditions are not completely satisfactory due to lack of efficacy and/or unwanted side effects of the compounds. The instant invention provides antisense compounds, preferably antisense compounds, for the prevention, amelioration, and /or treatment of pulmonary inflammation and/or airway hyperresponsiveness. As used herein, the term "prevention" means to delay or forestall onset or development of a condition or disease for a period of time from hours to days, preferably weeks to months. As used herein, the term "amelioration" means a lessening of at least one indicator of the severity of a condition or disease. The severity of indicators may be determined by subjective or objective measures which are known to those skilled in the art. As used herein, "treatment" means to administer a composition of the invention to effect an alteration or improvement of the disease or condition. Prevention, amelioration, and/or treatment may require administration of multiple doses at regular intervals, or prior to exposure to an agent (e.g., an allergen) to alter the course of the condition or disease. Moreover, a single agent may be used in a single individual for each prevention, amelioration, and treatment of a condition or disease, sequentially or concurrently. In a preferred method of the instant invention, the ASOs are delivered by aerosol for topical delivery to the respiratory tract, thereby limiting systemic exposure and reducing potential side effects.

### Overview

Disclosed herein are antisense compounds, including antisense oligonucleotides and other antisense compounds for use in modulating the expression of nucleic acid molecules encoding STAT 6. This is accomplished by providing antisense compounds that hybridize with one or more target nucleic acid molecules encoding STAT 6. As used herein, the terms "target nucleic acid" and "nucleic acid molecule encoding STAT 6 " have been used for convenience to encompass RNA (including pre-mRNA and mRNA or portions thereof) transcribed from DNA encoding STAT 6, and also cDNA derived from such RNA. In a preferred embodiment, the target nucleic acid is an mRNA encoding STAT 6.

The principle behind antisense technology is that an antisense compound hybridizes to a target nucleic acid to modulate gene expression activities such as transcription or translation. This sequence specificity makes antisense compounds extremely attractive for therapeutics to selectively modulate the expression of genes involved in disease, as well as tools for target validation and gene functional analysis. Although not limited by mechanism of action, the compounds of the instant invention are proposed to work by an antisense, non-autocatalytic mechanism.

### Target Nucleic Acids

"Targeting" an antisense compound to a particular target nucleic acid molecule can be a multistep process. The process usually begins with the identification of a target nucleic acid whose expression is to be modulated. For example, the target nucleic acid can be a cellular gene (or mRNA transcribed from the gene) whose expression is associated with a particular disorder or disease state, or a nucleic acid molecule from an infectious agent. As disclosed herein, the target nucleic acid encodes STAT 6.

### Variants

It is also known in the art that alternative RNA transcripts can be produced from the same genomic region of DNA. These alternative transcripts are generally known as "variants." More specifically, "pre-mRNA variants" are transcripts produced from the same genomic DNA that differ from other transcripts produced from the same genomic DNA in either their start or stop position and contain both intronic and exonic sequence. Variants can result in mRNA variants including, but not limited to, those with alternate splice junctions, or alternate initiation and termination codons. Variants in genomic and mRNA sequences can result in disease. Antisense compounds targeted to such variants are within the scope of the instant invention.

### Target Names, Synonyms, Features

In accordance with the present invention are compositions and methods for modulating the expression of STAT 6 (also known as IL4-STAT). There are also a number of isoforms of STAT 6 including STAT 6a (the main mRNA), STAT 6b, STAT 6c, STAT 6d, and STAT 6e. Table 1 lists the GenBank accession numbers of sequences corresponding to nucleic acid molecules encoding STAT 6 (nt = nucleotide), the date the version of the sequence was entered in GenBank, the isoform if not representing the main mRNA, and the corresponding SEQ ID NO in the instant application, when assigned, each of which is incorporated herein by reference.

**Table 1**

| **Gene Targets** | | | | |
|---|---|---|---|---|
| **Species** | **Genbank #** | **Genbank Date** | **STAT 6 Isoform** | **SEQ ID NO** |
| Human | NM_003153.1 | 24- MAR-1999 | | |
| Human | BC005823.1 | 4-APR-2001 | | |
| Human | AC018673.4, nt 157501-174000 | 30-NOV-2000 | | |
| Human | BE972840.1 | 4-OCT-2000 | d | |
| Human | BF902909.1 | 18-JAN-2001 | e | |
| Human | NM_003153.3 | 1-OCT-2002 | | 1 |
| Human | AR204914.1 | 20-JUN-2002 | b | |
| Human | AR204915.1 | 20-JUN-2002 | c | |
| Human | AF067572.1 | 25-OCT-1998 | | |
| Human | AF067573.1 | 25-OCT-1998 | | |
| Human | AF067574.1 | 25-OCT-1998 | | |
| Human | AF067575.1 | 25-OCT-1998 | | |
| Mouse | NM 009284.1 | 6-JAN-2000 | | |
| Mouse | BY723237.1 | 17-DEC-2002 | | |
| Mouse | BC029318.1 | 19-NOV-2003 | | |

### Modulation of Target Expression

Modulation of expression of a target nucleic acid can be achieved through alteration of any number of nucleic acid (DNA or RNA) functions. "Modulation" means a perturbation of function, for example, either an increase (stimulation or induction) or a decrease (inhibition or reduction) in expression. As another example, modulation of expression can include perturbing splice site selection of pre-mRNA processing. "Expression" includes all the functions by which a gene's coded information is converted into structures present and operating in a cell. These structures include the products of transcription and translation. "Modulation of expression" means the perturbation of such functions. The functions of RNA to be modulated can include translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. One result of such interference with target nucleic acid function is modulation of the expression of STAT 6. Thus, in one embodiment modulation of expression can mean increase or decrease in target RNA or protein levels. In another embodiment modulation of expression can mean a decrease or increase of one or more RNA splice products, or a change in the ratio of two or more splice products.

The effect of antisense compounds of the present invention on target nucleic acid expression can be tested in any of a variety of cell types provided that the target nucleic acid is present at measurable levels. The effect of antisense compounds of the present invention on target nucleic acid expression can be routinely determined using, for example, PCR or Northern blot analysis. Cell lines are derived from both normal tissues and cell types and from cells associated with various disorders (e.g. hyperproliferative disorders). Cell lines derived from multiple tissues and species can be obtained from American Type Culture Collection (ATCC, Manassas, VA) and other public sources, and are well known to those skilled in the art. Primary cells, or those cells which are isolated from an animal and not subj ected to continuous culture, can be prepared according to methods known in the art, or obtained from various commercial suppliers. Additionally, primary cells include those obtained from donor human subjects in a clinical setting (i.e. blood donors, surgical patients). Primary cells prepared by methods known in the art.

### Assaying Modulation of Expression

Modulation of STAT 6 expression can be assayed in a variety of ways known in the art. STAT 6 mRNA levels can be quantitated by, e.g., Northern blot analysis, competitive polymerase chain reaction (PCR), or real-time PCR. RNA analysis can be performed on total cellular RNA or poly(A)+ mRNA by methods known in the art. Methods of RNA isolation are taught in, for example, Ausubel, F.M. et al., Current Protocols in Molecular Biology, Volume 1, pp. 4.1.1-4.2.9 and 4.5.1-4.5.3, John Wiley & Sons, Inc., 1993.

Northern blot analysis is routine in the art and is taught in, for example, Ausubel, F.M. et al., Current Protocols in Molecular Biology, Volume 1, pp. 4.2.1-4.2.9, John Wiley & Sons, Inc., 1996. Real-time quantitative (PCR) can be conveniently accomplished using the commercially available ABI PRISM™ 7700 Sequence Detection System, available from PE-Applied Biosystems, Foster City, CA and used according to manufacturer's instructions. The method of analysis of modulation of RNA levels is not a limitation of the instant invention.

Levels of a protein encoded by STAT 6 can be quantitated in a variety of ways well known in the art, such as immunoprecipitation, Western blot analysis (immunoblotting), ELISA or fluorescence-activated cell sorting (FACS). Antibodies directed to a protein encoded by STAT 6 can be identified and obtained from a variety of sources, such as the MSRS catalog of antibodies (Aerie Corporation, Birmingham, MI), or can be prepared via conventional antibody generation methods. Methods for preparation of polyclonal antisera are taught in, for example, Ausubel, F.M. et al., Current Protocols in Molecular Biology, Volume 2, pp. 11.12.1-11.12.9, John Wiley & Sons, Inc., 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997.

### Active Target Segments

The locations on the target nucleic acid defined by having one or more active antisense compounds targeted thereto are referred to as "active target segments." When an active target segment is defined by multiple antisense compounds, the compounds are preferably separated by no more than about 50 nucleotides on the target sequence, more preferably no more than about 10 nucleotides on the target sequence, even more preferably the compounds are contiguous, most preferably the compounds are overlapping. There may be substantial variation in activity (e.g., as defined by percent inhibition) of the antisense compounds within an active target segment. Active antisense compounds are those that modulate the expression of their target RNA in the methods described herein. Active antisense compounds inhibit expression of their target RNA at least about 50%. In a preferred embodiment, at least about 50%, of the oligonucleotides targeted to the active target segment modulate expression of their target RNA at least 65%. In a more preferred embodiment, the level of inhibition required to define an active antisense compound is defined based on the results from the screen used to defme the active target segments.

### Hybridization

As used herein, "hybridization" means the pairing of complementary strands of antisense compounds to their target sequence. While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is complementary to the natural nucleobases thymidine and uracil which pair through the formation of hydrogen bonds. The natural base guanine is complementary to the natural bases cytosine and 5-methyl cytosine. Hybridization can occur under varying circumstances.

An antisense compound is specifically hybridizable when there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays.

As used herein, "stringent hybridization conditions" or "stringent conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated.

### Complementarity

"Complementarity," as used herein, refers to the capacity for precise pairing between two nucleobases on either two oligomeric compound strands or an antisense compound with its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be a complementary position.

"Complementarity" can also be viewed in the context of an antisense compound and its target, rather than in a base by base manner. The antisense compound and the further DNA or RNA are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the antisense compound and a target nucleic acid. One skilled in the art recognizes that the inclusion of mismatches is possible without eliminating the activity of the antisense compound. The invention is therefore directed to those antisense compounds that may contain up to about 20% nucleotides that disrupt base pairing of the antisense compound to the target. Preferably the compounds contain no more than about 15%, more preferably not more than about 10%, most preferably not more than 5% or no mismatches. The remaining nucleotides do not disrupt hybridization (e.g., universal bases).

### Identity

Antisense compounds, or a portion thereof, may have a defined percent identity to a SEQ ID NO, or a compound having a specific Isis number. As used herein, a sequence is identical to the sequence disclosed herein if it has the same nucleobase pairing ability. For example, a RNA which contains uracil in place of thymidine in the disclosed sequences of the instant invention would be considered identical as they both pair with adenine. This identity may be over the entire length of the oligomeric compound, or in a portion of the antisense compound (e.g., nucleobases 1-20 of a 27-mer may be compared to a 20-mer to determine percent identity of the oligomeric compound to the SEQ ID NO.) It is understood by those skilled in the art that an antisense compound need not have an identical sequence to those described herein to function similarly to the antisense compound described herein. Shortened versions of antisense compound taught herein, or non-identical versions of the antisense compound taught herein fall within the scope of the invention. Non-identical versions are those wherein each base does not have the same pairing activity as the antisense compounds disclosed herein. Bases do not have the same pairing activity by being shorter or having at least one abasic site. Alternatively, a non-identical version can include at least one base replaced with a different base with different pairing activity (e.g., G can be replaced by C, A, or T). Percent identity is calculated according to the number of bases that have identical base pairing corresponding to the SEQ ID NO or antisense compound to which it is being compared. The non-identical bases may be adjacent to each other, dispersed through out the oligonucleotide, or both.

For example, a 16-mer having the same sequence as nucleobases 2-17 of a 20-mer is 80% identical to the 20-mer. Alternatively, a 20-mer containing four nucleobases not identical to the 20-mer is also 80% identical to the 20-mer. A 14-mer having the same sequence as nucleobases 1-14 of an 18-mer is 78% identical to the 18-mer. Such calculations are well within the ability of those skilled in the art.

The percent identity is based on the percent of nucleobases in the original sequence present in a portion of the modified sequence. Therefore, a 30 nucleobase antisense compound comprising the full sequence of the complement of a 20 nucleobase active target segment would have a portion of 100% identity with the complement of the 20 nucleobase active target segment, while further comprising an additional 10 nucleobase portion. In the context of the invention, the complement of an active target segment may constitute a single portion. In a preferred embodiment, the oligonucleotides of the instant invention are at least about 80%, more preferably at least about 85%, even more preferably at least about 90%, most prefereably at least 95% identical to at least a portion of the complement of the active target segments presented herein.

It is well known by those skilled in the art that it is possible to increase or decrease the length of an antisense compound and/or introduce mismatch bases without eliminating activity. For example, in Woolf et al. (Proc. Natl. Acad. Sci. USA 89:7305-7309, 1992, incorporated herein by reference), a series of ASOs 13-25 nucleobases in length were tested for their ability to induce cleavage of a target RNA. ASOs 25 nucleobases in length with 8 or 11 mismatch bases near the ends of the ASOs were able to direct specific cleavage of the target mRNA, albeit to a lesser extent than the ASOs that contained no mismatches. Similarly, target specific cleavage was achieved using a 13 nucleobase ASOs, including those with 1 or 3 mismatches. Maher and Dolnick (Nuc. Acid. Res. 16:3341-3358,1988, incorporated herein by reference) tested a series of tandem 14 nucleobase ASOs, and a 28 and 42 nucleobase ASOs comprised of the sequence of two or three of the tandem ASOs, respectively, for their ability to arrest translation of human DHFR in a rabbit reticulocyte assay. Each of the three 14 nucleobase ASOs alone were able to inhibit translation, albeit at a more modest level than the 28 or 42 nucleobase ASOs. It is understood that antisense compounds of the instant invention can vary in length and percent complementarity to the target provided that they maintain the desired activity. Methods to determine desired activity are disclosed herein and well known to those skilled in the art.

### Therapeutics

Antisense compounds of the invention can be used to modulate the expression of STAT 6 in an animal, such as a human. In one non-limiting embodiment, the methods comprise the step of administering to said animal in need of therapy for a disease or condition associated with STAT 6 an effective amount of an antisense compound that inhibits expression of STAT 6. A disease or condition associated with STAT 6 includes, but is not limited to, pulmonary inflammation and airway hyperresponsiveness. In one embodiment, the antisense compounds of the present invention effectively inhibit the levels or function of STAT 6 RNA. Because reduction in STAT 6 mRNA levels can lead to alteration in STAT 6 protein products of expression as well, such resultant alterations can also be measured. Antisense compounds of the present invention that effectively inhibit the level or function of STAT 6 RNA or protein products of expression are considered active antisense compounds. In one embodiment, the antisense compounds of the invention inhibit the expression of STAT 6 causing a reduction of RNA, preferably in target cells or tissues, by at least 10%, by at least 20%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100%.

For example, the reduction of the expression of STAT 6 can be measured in a bodily fluid, which may or may not contain cells; tissue; or organ of the animal. Methods of obtaining samples for analysis, such as body fluids (e.g., sputum, serum), tissues (e.g., biopsy), or organs, and methods of preparation of the samples to allow for analysis are well known to those skilled in the art. Methods for analysis of RNA and protein levels are discussed above and are well known to those skilled in the art. The effects of treatment can be assessed by measuring biomarkers associated with the target gene expression in the aforementioned fluids, tissues or organs, collected from an animal contacted with one or more compounds of the invention, by routine clinical methods known in the art. These biomarkers include but are not limited to: liver transaminases, bilirubin, albumin, blood urea nitrogen, creatine and other markers of kidney and liver function; interleukins, tumor necrosis factors, intracellular adhesion molecules, C-reactive protein, chemokines, cytokines, and other markers of inflammation.

The antisense compounds of the present invention can be utilized in pharmaceutical compositions by adding an effective amount of a compound to a suitable pharmaceutically acceptable diluent or carrier. Acceptable carriers and dilutents are well known to those skilled in the art. Selection of a dilutent or carrier is based on a number of factors, including, but not limited to, the solubility of the compound and the route of administration. Such considerations are well understood by those skilled in the art. In one aspect, the antisense compounds of the present invention inhibit the expression of STAT 6. The compounds of the invention can also be used in the manufacture of a medicament for the treatment of diseases and disorders related to STAT 6 expression.

Methods whereby bodily fluids, organs or tissues are contacted with an effective amount of one or more of the antisense compounds or compositions of the invention are also contemplated. Bodily fluids, organs or tissues can be contacted with one or more of the compounds of the invention resulting in modulation of STAT 6 expression in the cells of bodily fluids, organs or tissues. An effective amount can be determined by monitoring the modulatory effect of the antisense compound or compounds or compositions on target nucleic acids or their products by methods routine to the skilled artisan.

Thus, provided herein is the use of an isolated single- or double-stranded antisense compound targeted to STAT 6 in the manufacture of a medicament for the treatment of a disease or disorder by means of the method described above. In a preferred embodiment, the antisense compound is a single stranded antisense compound. Such antisense compounds can function by any of a number of non-autocatalytic mechanisms incluing by the action of RNases (e.g., RNaseH) or modulation of splicing. Alternative antisense mechanisms (e.g., RNAi) can be promoted by the inclusion of a second, complementary strand to the antisense compound and/or inclusion of specific chemical modifications which are known to those skilled in the art.

### Kits, Research Reagents, and Diagnostics

The antisense compounds of the present invention can be utilized for diagnostics, and as research reagents and kits. Furthermore, antisense compounds, which are able to inhibit gene expression with specificity, are often used by those of ordinary skill to elucidate the function of particular genes or to distinguish between functions of various members of a biological pathway.

For use in kits and diagnostics, the antisense compounds of the present invention, either alone or in combination with other compounds or therapeutics, can be used as tools in differential and/or combinatorial analyses to elucidate expression patterns of a portion or the entire complement of genes expressed within cells and tissues. Methods of gene expression analysis are well known to those skilled in the art.

### Compounds

The term "oligomeric compound" refers to a polymeric structure capable of hybridizing to a region of a nucleic acid molecule. Generally, oligomeric compounds comprise a plurality of monomeric subunits linked together by internucleoside linking groups and/or internucleoside linkage mimetics. Each of the monomeric subunits comprises a sugar, abasic sugar, modified sugar, or a sugar mimetic, and except for the abasic sugar includes a nucleobase, modified nucleobase or a nucleobase mimetic. Preferred monomeric subunits comprise nucleosides and modified nucleosides.

An "antisense compound" or "antisense oligomeric compound" refers to an oligomeric compound that is at least partially complementary to the region of a target nucleic acid molecule to which it hybridizes and which modulates (increases or decreases) its expression. This term includes oligonucleotides, oligonucleosides, oligonucleotide analogs, oligonucleotide mimetics, antisense compounds, antisense oligomeric compounds, and chimeric combinations of these. Consequently, while all antisense compounds can be said to be oligomeric compounds, not all oligomeric compounds are antisense compounds. An "antisense oligonucleotide" is an antisense compound that is a nucleic acid-based oligomer. An antisense oligonucleotide can, in some cases, include one or more chemical modifications to the sugar, base, and/or internucleoside linkages. Nonlimiting examples of antisense compounds include primers, probes, antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. Antisense double-stranded compounds can be two strands hybridized to form double-stranded compounds or a single strand with sufficient self complementarity to allow for hybridization and formation of a fully or partially double-stranded compound. The compounds of the instant invention are not auto-catalytic. As used herein, "auto-catalytic" means a compound has the ability to promote cleavage of the target RNA in the absence of accessory factors, e.g. proteins.

In one embodiment of the invention, the antisense compound comprises a single stranded oligonucleotide. In some embodiments of the invention the antisense compound contains chemical modifications. In a preferred embodiment, the antisense compound is a single stranded, chimeric oligonucleotide wherein the modifications of sugars, bases, and internucleoside linkages are independently selected.

The antisense compounds in accordance with this invention may comprise an antisense compound from about 12 to about 35 nucleobases (i.e. from about 12 to about 35 linked nucleosides). In other words, a single-stranded compound of the invention comprises from about 12 to about 35 nucleobases, and a double-stranded antisense compound of the invention (such as a siRNA, for example) comprises two strands, each of which is independently from about 12 to about 35 nucleobases. This includes oligonucleotides 15 to 35 and 16 to 35 nucleobases in length. Contained within the antisense compounds of the invention (whether single or double stranded and on at least one strand) are antisense portions. The "antisense portion" is that part of the antisense compound that is designed to work by one of the aforementioned antisense mechanisms. One of ordinary skill in the art will appreciate that about 12 to about 35 nucleobases includes 12, 13, 14, 15, 16, 17, 18, 19, 20, 21, 22, 23, 24, 25, 26, 27, 28, 29, 30, 31, 32, 33, 34, or 35 nucleobases.

Antisense compounds about 12 to 35 nucleobases in length, preferably about 15 to 35 nucleobases in length, comprising a stretch of at least eight (8), preferably at least 12, more preferably at least 15 consecutive nucleobase targeted to the active target regions are considered to be suitable antisense compounds as well.

Modifications can be made to the antisense compounds of the instant invention and may include conjugate groups attached to one of the termini, selected nucleobase positions, sugar positions or to one of the internucleoside linkages. Possible modifications include, but are not limited to, 2'-fluoro (2'-F), 2'-OMethyl (2'-OMe), 2'-Methoxy ethoxy (2'-MOE) sugar modifications, inverted abasic caps, deoxynucleobases, and bicyclice nucleobase analogs such as locked nucleic acids (including LNA) and ENA.

In one embodiment of the invention, double-stranded antisense compounds encompass short interfering RNAs (siRNAs). As used herein, the term "siRNA" is defined as a double-stranded compound having a first and second strand, each strand having a central portion and two independent terminal portions. The central portion of the first strand is complementary to the central portion of the second strand, allowing hybridization of the strands. The terminal portions are independently, optionally complementary to the corresponding terminal portion of the complementary strand. The ends of the strands may be modified by the addition of one or more natural or modified nucleobases to form an overhang.

Each strand of the siRNA duplex may be from about 12 to about 35 nucleobases. In a preferred embodiment, each strand of the siRNA duplex is about 17 to about 25 nucleobases. The two strands may be fully complementary (i.e., form a blunt ended compound), or include a 5' or 3' overhang on one or both strands. Double-stranded compounds can be made to include chemical modifications as discussed herein. Structures of siRNAs are well known to those skilled in the art (see e.g., Guo and Kempheus, Cell, 1995, 81, 611-620; Montgomery et al.., Proc. Natl. Acad. Sci. USA, 1998, 95, 15502-15507; and Fire et al., Nature, 1998, 391, 806-811).

### Chemical Modifications

As is known in the art, a nucleoside is a base-sugar combination. The base portion of the nucleoside is normally a heterocyclic base (sometimes referred to as a "nucleobase" or simply a "base"). The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. In forming oligonucleotides, the phosphate groups covalently link adjacent nucleosides to one another to form a linear polymeric compound. Within oligonucleotides, the phosphate groups are commonly referred to as forming the internucleoside backbone of the oligonucleotide. The normal linkage or backbone of RNA and DNA is a 3' to 5' phosphodiester linkage. It is often preferable to include chemical modifications in oligonucleotides to alter their activity. Chemical modifications can alter oligonucleotide activity by, for example: increasing affinity of an antisense oligonucleotide for its target RNA, increasing nuclease resistance, and/or altering the pharmacokinetics of the oligonucleotide. The use of chemistries that increase the affinity of an oligonucleotide for its target can allow for the use of shorter oligonucleotide compounds.

The term "nucleobase" or "heterocyclic base moiety" as used herein, refers to the heterocyclic base portion of a nucleoside. In general, a nucleobase is any group that contains one or more atom or groups of atoms capable of hydrogen bonding to a base of another nucleic acid. In addition to "unmodified" or "natural" nucleobases such as the purine nucleobases adenine (A) and guanine (G), and the pyrimidine nucleobases thymine (T), cytosine (C) and uracil (U), many modified nucleobases or nucleobase mimetics known to those skilled in the art are amenable to the present invention. The terms modified nucleobase and nucleobase mimetic can overlap but generally a modified nucleobase refers to a nucleobase that is fairly similar in structure to the parent nucleobase, such as for example a 7-deaza purine, a 5-methyl cytosine, or a G-clamp , whereas a nucleobase mimetic would include more complicated structures, such as for example a tricyclic phenoxazine nucleobase mimetic. Methods for preparation of the above noted modified nucleobases are well known to those skilled in the art.

Antisense compounds of the present invention may also contain one or more nucleosides having modified sugar moieties. The furanosyl sugar ring of a nucleoside can be modified in a number of ways including, but not limited to, addition of a substituent group, bridging of two non-geminal ring atoms to form a bicyclic nucleic acid (BNA) and substitution of an atom or group such as -S-, -N(R)- or -C(R₁)(R₂) for the ring oxygen at the 4'-position. Modified sugar moieties are well known and can be used to alter, typically increase, the affinity of the antisense compound for its target and/or increase nuclease resistance. A representative list of preferred modified sugars includes but is not limited to bicyclic modified sugars (BNA's), including LNA and ENA (4'-(CH₂)₂₋O-2' bridge); and substituted sugars, especially 2'-substituted sugars having a 2'-F, 2'-OCH₂ or a 2'-O(CH₂)₂-OCH₃ substituent group. Sugars can also be replaced with sugar mimetic groups among others. Methods for the preparations of modified sugars are well known to those skilled in the art.

The present invention includes internucleoside linking groups that link the nucleosides or otherwise modified monomer units together thereby forming an antisense compound. The two main classes of internucleoside linking groups are defined by the presence or absence of a phosphorus atom. Representative phosphorus containing internucleoside linkages include, but are not limited to, phosphodiesters, phosphotriesters, methylphosphonates, phosphoramidate, and phosphorothioates. Representative non-phosphorus containing internucleoside linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)2-O-); and N,N'-dimethylhydrazine (-CH₂-N(CH₃)-N(CH₃)-). Antisense compounds having non-phosphorus internucleoside linking groups are referred to as oligonucleosides. Modified internucleoside linkages, compared to natural phosphodiester linkages, can be used to alter, typically increase, nuclease resistance of the antisense compound. Internucleoside linkages having a chiral atom can be prepared racemic, chiral, or as a mixture. Representative chiral internucleoside linkages include, but are not limited to, alkylphosphonates and phosphorothioates. Methods of preparation of phosphorous-containing and non-phosphorous-containing linkages are well known to those skilled in the art.

As used herein the term "mimetic" refers to groups that are substituted for a sugar, a nucleobase, and/ or internucleoside linkage. Generally, a mimetic is used in place of the sugar or sugar-internucleoside linkage combination, and the nucleobase is maintained for hybridization to a selected target. Representative examples of a sugar mimetic include, but are not limited to, cyclohexenyl or morpholino. Representative examples of a mimetic for a sugar-internucleoside linkage combination include, but are not limited to, peptide nucleic acids (PNA) and morpholino groups linked by uncharged achiral linkages. In some instances a mimetic is used in place of the nucleobase. Representative nucleobase mimetics are well known in the art and include, but are not limited to, tricyclic phenoxazine analogs and universal bases (Berger et al., Nuc Acid Res. 2000, 28:2911-14, incorporated herein by reference). Methods of synthesis of sugar, nucleoside and nucleobase mimetics are well known to those skilled in the art.

As used herein the term "nucleoside" includes, nucleosides, abasic nucleosides, modified nucleosides, and nucleosides having mimetic bases and/or sugar groups.

In the context of this invention, the term "oligonucleotide" refers to an oligomeric compound which is an oligomer or polymer of ribonucleic acid (RNA) or deoxyribonucleic acid (DNA). This term includes oligonucleotides composed of naturally- and non-naturally-occurring nucleobases, sugars and covalent internucleoside linkages, possibly further including non-nucleic acid conjugates.

The present invention provides compounds having reactive phosphorus groups useful for forming internucleoside linkages including for example phosphodiester and phosphorothioate internucleoside linkages. Methods of preparation and/or purification of precursors or antisense compounds of the instant invention are not a limitation of the compositions or methods of the invention. Methods for synthesis and purification of DNA, RNA, and the antisense compounds of the instant invention are well known to those skilled in the art.

As used herein the term "chimeric antisense compound" refers to an antisense compound, having at least one sugar, nucleobase and/or internucleoside linkage that is differentially modified as compared to the other sugars, nucleobases and internucleoside linkages within the same oligomeric compound. The remainder of the sugars, nucleobases and internucleoside linkages can be independently modified or unmodified. In general a chimeric oligomeric compound will have modified nucleosides that can be in isolated positions or grouped together in regions that will define a particular motif. Any combination of modifications and or mimetic groups can comprise a chimeric oligomeric compound of the present invention.

Chimeric oligomeric compounds typically contain at least one region modified so as to confer increased resistance to nuclease degradation, increased cellular uptake, and/or increased binding affinity for the target nucleic acid. An additional region of the oligomeric compound may serve as a substrate for enzymes capable of cleaving RNA:DNA or RNA:RNA hybrids. By way of example, RNase H is a cellular endonuclease that cleaves the RNA strand of an RNA:DNA duplex. Activation of RNase H, therefore, results in cleavage of the RNA target, thereby greatly enhancing the efficiency of inhibition of gene expression. Consequently, comparable results can often be obtained with shorter oligomeric compounds when chimeras are used, compared to for example phosphorothioate deoxyoligonucleotides hybridizing to the same target region. Cleavage of the RNA target can be routinely detected by gel electrophoresis and, if necessary, associated nucleic acid hybridization techniques known in the art.

Certain chimeric as well as non-chimeric oligomeric compounds can be further described as having a particular motif. As used in the present invention the term "motif'' refers to the orientation of modified sugar moieties and/or sugar mimetic groups in an antisense compound relative to like or differentially modified or unmodified nucleosides. As used in the present invention, the terms "sugars", "sugar moieties" and "sugar mimetic groups' are used interchangeably. Such motifs include, but are not limited to, gapped motifs, alternating motifs, fully modified motifs, hemimer motifs, blockmer motifs, and positionally modified motifs. The sequence and the structure of the nucleobases and type of internucleoside linkage is not a factor in determining the motif of an antisense compound.

As used in the present invention the term "gapped motif'' refers to an antisense compound comprising a contiguous sequence of nucleosides that is divided into 3 regions, an internal region (gap) flanked by two external regions (wings). The regions are differentiated from each other at least by having differentially modified sugar groups that comprise the nucleosides. In some embodiments, each modified region is uniformly modified (e.g. the modified sugar groups in a given region are identical); however, other motifs can be applied to regions. For example, the wings in a gapmer could have an alternating motif. The nucleosides located in the gap of a gapped antisense compound have sugar moieties that are different than the modified sugar moieties in each of the wings. In a preferred embodiment of the invention, the antisense compounds are 5-10-5 MOE gapmers having a 2'-MOE modifications on nucleobases 1-5 and 16-20, all cytosines are 5MeC, and a full phosphorothioate backbone.

As used in the present invention the term "alternating motif' refers to an antisense compound comprising a contiguous sequence of nucleosides comprising two differentially sugar modified nucleosides that alternate for essentially the entire sequence of the antisense compound, or for essentially the entire sequence of a region of an antisense compound.

As used in the present invention the term "fully modified motif' refers to an antisense compound comprising a contiguous sequence of nucleosides wherein essentially each nucleoside is a sugar modified nucleoside having uniform modification.

As used in the present invention the term "hemimer motif' refers to a sequence of nucleosides that have uniform sugar moieties (identical sugars, modified or unmodified) and wherein one of the 5'-end or the 3'-end has a sequence of from 2 to 12 nucleosides that are sugar modified nucleosides that are different from the other nucleosides in the hemimer modified antisense compound.

As used in the present invention the term "blockmer motif' refers to a sequence of nucleosides that have uniform sugars (identical sugars, modified or unmodified) that is internally interrupted by a block of sugar modified nucleosides that are uniformly modified and wherein the modification is different from the other nucleosides. Methods of preparation of chimeric oligonucleotide compounds are well known to those skilled in the art.

As used in the present invention the term "positionally modified motif' comprises all other motifs. Methods of preparation of positionally modified oligonucleotide compounds are well known to those skilled in the art.

The compounds described herein contain one or more asymmetric centers and thus give rise to enantiomers, diastereomers, and other stereoisomeric configurations that may be defined, in terms of absolute stereochemistry, as (R) or (S), α or β, or as (D) or (L) such as for amino acids et al. The present invention is meant to include all such possible isomers, as well as their racemic and optically pure forms.

In one aspect of the present invention antisense compounds are modified by covalent attachment of one or more conjugate groups. Conjugate groups may be attached by reversible or irreversible attachments. Conjugate groups may be attached directly to antisense compounds or by use of a linker. Linkers may be mono- or bifunctional linkers. Such attachment methods and linkers are well known to those skilled in the art. In general, conjugate groups are attached to antisense compounds to modify one or more properties. Such considerations are well known to those skilled in the art.

### Oligomer Synthesis

Oligomerization of modified and unmodified nucleosides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Antisense compounds of the present invention can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

### Oligomer Purification and Analysis

Methods of oligonucleotide purification and analysis are known to those skilled in the art. Analysis methods include capillary electrophoresis (CE) and electrospray-mass spectroscopy. Such synthesis and analysis methods can be performed in multi-well plates. The method of the invention is not limited by the method of oligomer purification.

### Salts, prodrugs and bioequivalents

The antisense compounds of the present invention comprise any pharmaceutically acceptable salts, esters, or salts of such esters, or any other functional chemical equivalent which, upon administration to an animal including a human, is capable of providing (directly or indirectly) the biologically active metabolite or residue thereof. Accordingly, for example, the disclosure is also drawn to prodrugs and pharmaceutically acceptable salts of the antisense compounds of the present invention, pharmaceutically acceptable salts of such prodrugs, and other bioequivalents.

The term "prodrug" indicates a therapeutic agent that is prepared in an inactive or less active form that is converted to an active form (i.e., drug) within the body or cells thereof by the action of endogenous enzymes, chemicals, and/or conditions. In particular, prodrug versions of the oligonucleotides of the invention are prepared as SATE ((S-acetyl-2-thioethyl) phosphate) derivatives according to the methods disclosed in WO 93/24510 or WO 94/26764. Prodrugs can also include antisense compounds wherein one or both ends comprise nucleobases that are cleaved (e.g., by incorporating phosphodiester backbone linkages at the ends) to produce the active compound.

The term "pharmaceutically acceptable salts" refers to physiologically and pharmaceutically acceptable salts of the compounds of the invention: i.e., salts that retain the desired biological activity of the parent compound and do not impart undesired toxicological effects thereto. Sodium salts of antisense oligonucleotides are useful and are well accepted for therapeutic administration to humans. In another embodiment, sodium salts of dsRNA compounds are also provided.

### Formulations

The antisense compounds of the invention may also be admixed, encapsulated, conjugated or otherwise associated with other molecules, molecule structures or mixtures of compounds.

The present invention also includes pharmaceutical compositions and formulations which include the antisense compounds of the invention. The pharmaceutical compositions of the present invention may be administered in a number of ways depending upon whether local or systemic treatment is desired and upon the area to be treated. In a preferred embodiment, administration is topical to the surface of the respiratory tract, particularly pulmonary, e.g., by nebulization, inhalation, or insufflation of powders or aerosols, by mouth and/or nose.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers, finely divided solid carriers, or both, and then, if necessary, shaping the product (e.g., into a specific particle size for delivery). In a preferred embodiment, the pharmaceutical formulations of the instant invention are prepared for pulmonary administration in an appropriate solvent, e.g., water or normal saline, possibly in a sterile formulation, with carriers or other agents to allow for the formation of droplets of the desired diameter for delivery using inhalers, nasal delivery devices, nebulizers, and other devices for pulmonary delivery. Alternatively, the pharmaceutical formulations of the instant invention may be formulated as dry powders for use in dry powder inhalers.

A "pharmaceutical carrier" or "excipient" can be a pharmaceutically acceptable solvent, suspending agent or any other pharmacologically inert vehicle for delivering one or more nucleic acids to an animal and are known in the art. The excipient may be liquid or solid and is selected, with the planned manner of administration in mind, so as to provide for the desired bulk, consistency, etc., when combined with a nucleic acid and the other components of a given pharmaceutical composition.

### Combinations

Compositions of the invention can contain two or more antisense compounds. In another related embodiment, compositions of the present invention can contain one or more antisense compounds, particularly oligonucleotides, targeted to a first nucleic acid and one or more additional antisense compounds targeted to a second nucleic acid target. Alternatively, compositions of the present invention can contain two or more antisense compounds targeted to different regions of the same nucleic acid target. Two or more combined compounds may be used together or sequentially. Compositions of the instant invention can also be combined with other non-antisense compound therapeutic agents.

### Nonlimiting disclosure and incorporation by reference

While certain compounds, compositions and methods of the present invention have been described with specificity in accordance with certain embodiments, the following examples serve only to illustrate the compounds of the invention and are not intended to limit the same. Each of the references, GenBank accession numbers, and the like recited in the present application is incorporated herein by reference in its entirety.

### Example 1: Transfection methods

### Cell types

The effect of antisense compounds on target nucleic acid expression was tested in the following cell types.

### T-24 cells:

The transitional cell bladder carcinoma cell line T-24 was obtained from the American Type Culture Collection (ATCC) (Manassas, VA). T-24 cells were routinely cultured in complete McCoy's 5A basal media (Invitrogen Life Technologies, Carlsbad, CA) supplemented with 10% fetal bovine serum (Invitrogen Life Technologies, Carlsbad, CA). Cells were routinely passaged by trypsinization and dilution when they reached approximately 90% confluence. Cells were seeded into 96-well plates (Falcon-Primaria #3872) at a density of approximately 4000-6000 cells/well for use in oligomeric compound transfection experiments.

### A549:

The human lung carcinoma cell line A549 was obtained from the American Type Culture Collection (Manassas, VA). A549 cells were routinely cultured in DMEM, high glucose (Invitrogen Life Technologies, Carlsbad, CA) supplemented with 10% fetal bovine serum, 100 units per ml penicillin, and 100 micrograms per ml streptomycin (Invitrogen Life Technologies, Carlsbad, CA). Cells were routinely passaged by trypsinization and dilution when they reached approximately 90% confluence. Cells were seeded into 96-well plates (Falcon-Primaria #3872) at a density of approximately 5000 cells/well for use in antisense compound transfection experiments.

### b.END:

The mouse brain endothelial cell line b.END was obtained from Dr. Werner Risau at the Max Plank Institute (Bad Nauheim, Germany). b.END cells were routinely cultured in DMEM, high glucose (Invitrogen Life Technologies, Carlsbad, CA) supplemented with 10% fetal bovine serum (Invitrogen Life Technologies, Carlsbad, CA). Cells were routinely passaged by trypsinization and dilution when they reached approximately 90% confluence. Cells were seeded into 96-well plates (Falcon-Primaria #353872, BD Biosciences, Bedford, MA) at a density of approximately 3000 cells/well for use in oligomeric compound transfection experiments.

### Treatment with antisense compounds

When cells reach appropriate confluency, they are treated with oligonucleotide using a transfection lipid and method, such as Lipofectin™ essentially by the manufacturer's instructions, as described.

Briefly, when cells reached 65-75% confluency, they were treated with oligonucleotide. Oligonucleotide was mixed with LIPOFECTIN™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTIN™ concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture was replaced with fresh culture medium. Cells were harvested 16-24 hours after oligonucleotide treatment.

Other transfection reagents and methods (e.g., electroporation) for delivery of oligonucleotides to the cell are well known. The method of delivery of oligonucleotide to the cells is not a limitation of the instant invention.

### Control oligonucleotides

Control oligonucleotides are used to determine the optimal antisense compound concentration for a particular cell line. Furthermore, when antisense compounds of the invention are tested in antisense compound screening experiments or phenotypic assays, control oligonucleotides are tested in parallel with compounds of the invention.

The concentration of oligonucleotide used varies from cell line to cell line. To determine the optimal oligonucleotide concentration for a particular cell line, the cells are treated with a positive control oligonucleotide at a range of concentrations. The concentration of positive control oligonucleotide that results in 80% inhibition of the target mRNA is then utilized as the screening concentration for new oligonucleotides in subsequent experiments for that cell line. If 80% inhibition is not achieved, the lowest concentration of positive control oligonucleotide that results in 60% inhibition of the target mRNA is then utilized as the oligonucleotide screening concentration in subsequent experiments for that cell line. If 60% inhibition is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 µM to 40 µM when the antisense oligonucleotide is transfected by electroporation.

### Example 2: Real-time Quantitative PCR Analysis of STAT 6 mRNA Levels

Quantitation of STAT 6 mRNA levels was accomplished by real-time quantitative PCR using the ABI PRISM™ 7600, 7700, or 7900 Sequence Detection System (PE-Apphed Biosystems, Foster City, CA) according to manufacturer's instructions.

Prior to quantitative PCR analysis, primer-probe sets specific to the target gene being measured were evaluated for their ability to be "multiplexed" with a GAPDH amplification reaction. After isolation the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR were normalized using either the expression level of GAPDH, a gene whose expression is constant, or by quantifying total RNA using RiboGreen^{™} (Molecular Probes, Inc. Eugene, OR). GAPDH expression was quantified by RT, real-time PCR, by being run simultaneously with the target, multiplexing, or separately. Total RNA was quantified using RiboGreen^{™} RNA quantification reagent (Molecular Probes, Inc. Eugene, OR).

170 µL of RiboGreen^{™} working reagent (RiboGreen^{™} reagent diluted 1:350 in 10mM Tris-HCl, 1 mM EDTA, pH 7.5) was pipetted into a 96-well plate containing 30 µL purified cellular RNA. The plate was read in a CytoFluor 4000 (PE Applied Biosystems) with excitation at 485nm and emission at 530nm.

The GAPDH PCR probes have JOE covalently linked to the 5' end and TAMRA or MGB covalently linked to the 3' end, where JOE is the fluorescent reporter dye and TAMRA or MGB is the quencher dye. In some cell types, primers and probe designed to a GAPDH sequence from a different species are used to measure GAPDH expression. For example, a human GAPDH primer and probe set is used to measure GAPDH expression in monkey-derived cells and cell lines.

Probes and primers for use in real-time PCR were designed to hybridize to target-specific sequences. Design of probes and primers is well with the ability of those skilled in the art. The target-specific PCR probes have FAM covalently linked to the 5' end and TAMRA or MGB covalently linked to the 3' end, where FAM is the fluorescent dye and TAMRA or MGB is the quencher dye.

### Example 3: Antisense inhibition of human STAT 6 expression by antisense compounds

A series of antisense compounds was designed to target different regions of human STAT 6 RNA, using published sequences or portions of published sequences as cited in Table 1, specifically GenBank number NM_003153.3 (SEQ ID NO: 1). A number antisense compounds taught in US Patent Publications US20040115634 and 20050239124, can also be mapped to SEQ ID NO: 1 and are shown in Table 2. In the inhibition studies, T-24 cells were treated with 100 nM of oligonucleotide using LIPOFECTIN™. Inhibition of mRNA target expression was determined using the RT-PCR method detailed above. The results are shown in Table 2.

**Table 2**

| **Inhibition of human STAT 6 mRNA levels by chimeric phosphorothioate oligonucleotides having 2'-MOE wings and a deoxy gap** | | | |
|---|---|---|---|
| **ISIS #** | **TARGET SITE** | **SEQUENCE** | **% INHIB** |
| 153765 | 2057 | agtgagcgaatggacaggtc | 96 |
| 153766 | 2589 | cgctgtcactggctggctca | 83 |
| 153767 | 127 | ttgatgatttctccagtgct | 92 |
| 153768 | 1046 | aggacttcatccagccggcc | 50 |
| 153769 | 1160 | cccaggaacctcaagcccaa | 68 |
| 153770 | 348 | gtcacccagaagatgccgca | 53 |
| 153771 | 2206 | tttccacggtcatcttgatg | 59 |
| 153772 | 485 | aagatggtgctcccctcccc | 34 |
| 153773 | 871 | gccgtttccaaatctggatc | 76 |
| 153774 | 835 | ctttggctgcctctagctct | 89 |
| 153775 | 1831 | gtttggtgaggtccaggaca | 89 |
| 153776 | 2427 | catctgcaggtgaggctcct | 85 |
| 153777 | 2782 | tggcccttaggtccatgtgg | 76 |
| 153778 | 2011 | ctatctgtggagagccatcc | 72 |
| 153779 | 1911 | attgagaagaaggctagtaa | 83 |
| 153780 | 498 | gctgatgttgcaagatgg | 78 |
| 153781 | 3125 | gccccatcaccctcagagag | 80 |
| 153782 | 523 | ccctctgatatatgctctca | 73 |
| 153783 | 1903 | gaaggctagtaacgtactgt | 84 |
| 153784 | 1925 | gttccgtcgggctcattgag | 92 |
| 153785 | 2585 | gtcactggctggctcaggca | 87 |
| 153786 | 1619 | ttcagagtttcacacatctt | 83 |
| 153787 | 185 | caggccccataggtctgtag | 88 |
| 153788 | 750 | tatcaagctgtgcagagaca | 80 |
| 153789 | 378 | caggaactcccagggctggc | 74 |
| 153790 | 3106 | gctctgtatgtgtgtgtgcg | 90 |
| 153791 | 2078 | agatcccggattcggtcccc | 88 |
| 153792 | 900 | cggtgcgccattccctgcca | 94 |
| 153793 | 2103 | gggatagagatttttgagct | 53 |
| 153794 | 252 | gatctgggacttggaggttg | 71 |
| 153795 | 2271 | tccaaggtcataagaaggca | 88 |
| 153796 | 1868 | atgatcagccggtcagacca | 84 |
| 153797 | 1311 | cccaggaatgctgttctcca | 88 |
| 153798 | 1050 | tctcaggacttcatccagcc | 6 |
| 153799 | 1777 | ccagcaggatctccttgttg | 82 |
| 153800 | 1266 | tccagtgctttctgctccag | 87 |
| 153801 | 434 | acagtgtctgaaagtagggc | 50 |
| 195427 | 145 | gctggccctgctagcacctc | 68 |
| 195428 | 264 | ccacagagacatgatctggg | 79 |
| 195429 | 647 | gtcttaaacttgagttcttc | 53 |
| 195430 | 824 | tctagctctccagtggtctc | 78 |
| 195431 | 1191 | ggccctgaccagcggaggct | 72 |
| 195432 | 1396 | cctctgtgacagactcagtg | 72 |
| 195433 | 1721 | tccatactgaggctgttgtc | 20 |
| 195434 | 1993 | cctggccccggatgacatgg | 53 |
| 195435 | 2258 | gaaggcaccatggtaggcat | 55 |
| 195436 | 2612 | ccaatccaagtgccctgagg | 70 |
| 195437 | 2805 | cagctgggatcaccaactgg | 49 |
| 195438 | 3050 | gtgtctcagagcctgaactt | 77 |
| 195439 | 14 | taagcagtggctgccccagc | 51 |
| 195440 | 29 | cctccctcttcagtgtaagc | 65 |
| 195441 | 3177 | agaagccttccatgccctaa | 83 |
| 195442 | 3222 | tatgttcctgcctatccgtc | 76 |
| 195443 | 3523 | caactaaggtgccagctata | 86 |
| 195444 | 3531 | tggtcatgcaactaaggtgc | 84 |
| 195445 | 3577 | atttgtgttgtcacgtaggc | 84 |
| 195446 | 3591 | tctcaccctcccaaatttgt | 48 |
| 195447 | 3621 | agcacacttgctgctgtctt | 74 |
| 195448 | 3771 | gccaggcctggacccagact | 60 |
| 195449 | 3827 | gggcaacagaaaagatgcag | 50 |
| 195453 | 1558 | ccatctcagagaaggcattg | 81 |

A series of antisense compounds was designed to target different regions of human STAT 6 RNA, using published sequences or portions of published sequences as cited in Table 1, specifically GenBank number NM_003153.3 (SEQ ID NO: 1). In the inhibition studies, A549 cells were treated with 50nM of oligonucleotide using LIPOFECTIN™. Inhibition of mRNA target expression was determined using the RT-PCR method detailed above. The results are shown in Tables 3 and 4.

**Table 3**

| **Inhibition of human STAT 6 mRNA levels by chimeric phosphorothioate oligonucleotides having 2'-MOE wings and a deoxy gap** | | | |
|---|---|---|---|
| **ISIS #** | **TARGET SITE** | **SEQUENCE (5' to 3')** | **% INHIB** |
| 369370 | 12 | AGCAGTGGCTGCCCCAGCCC | 59 |
| 369371 | 20 | TCAGTGTAAGCAGTGGCTGC | 66 |
| 369372 | 27 | TCCCTCTTCAGTGTAAGCAG | 66 |
| 369373 | 154 | GAGTTCAAGGCTGGCCCTGC | 75 |
| 369374 | 248 | TGGGACTTGGAGGTTGCCTC | 52 |
| 369375 | 253 | TGATCTGGGACTTGGAGGTT | 64 |
| 369376 | 258 | AGACATGATCTGGGACTTGG | 80 |
| 369377 | 263 | CACAGAGACATGATCTGGGA | 70 |
| 369378 | 268 | GACCCCACAGAGACATGATC | 29 |
| 369379 | 272 | ACCAGACCCCACAGAGACAT | 35 |
| 369380 | 279 | CTTGGAGACCAGACCCCACA | 48 |
| 369381 | 284 | GGCATCTTGGAGACCAGACC | 69 |
| 369382 | 351 | CCAGTCACCCAGAAGATGCC | 14 |
| 369383 | 356 | TCCAGCCAGTCACCCAGAAG | 37 |
| 369384 | 426 | TGAAAGTAGGGCACTAGCCA | 51 |
| 369385 | 431 | GTGTCTGAAAGTAGGGCACT | 77 |
| 369386 | 439 | GCTGGACAGTGTCTGAAAGT | 72 |
| 369387 | 497 | CTGATGTGTTGCAAGATGGT | 74 |
| 369388 | 525 | GTCCCTCTGATATATGCTCT | 76 |
| 369389 | 546 | AGTGGCCACCAGCTTCAGGG | 51 |
| 369390 | 551 | CTGAAAGTGGCCACCAGCTT | 64 |
| 369391 | 561 | AAGTATTTGTCTGAAAGTGG | 74 |
| 369392 | 567 | TCCTTGAAGTATTTGTCTGA | 48 |
| 369393 | 615 | GAAAGGCATTGGCAAGTGGC | 81 |
| 369394 | 620 | CAGTGGAAAGGCATTGGCAA | 80 |
| 369395 | 630 | TTCCTGCTTCCAGTGGAAAG | 70 |
| 369396 | 641 | AACTTGAGTTCTTCCTGCTT | 75 |
| 369397 | 740 | TGCAGAGACACTTGGCCAGC | 53 |
| 369398 | 760 | CAGGAGTTTCTATCAAGCTG | 77 |
| 369399 | 765 | ATTAGCAGGAGTTTCTATCA | 25 |
| 369400 | 770 | GTCCCATTAGCAGGAGTTTC | 70 |
| 369401 | 775 | GCCCAGTCCCATTAGCAGGA | 75 |
| 369402 | 780 | ACTTGGCCCAGTCCCATTAG | 44 |
| 369403 | 786 | GGCCTCACTTGGCCCAGTCC | 55 |
| 369404 | 792 | GGCCAGGGCCTCACTTGGCC | 38 |
| 369405 | 857 | TGGATCCTCTTCAGCACTAG | 32 |
| 369406 | 862 | AAATCTGGATCCTCTTCAGC | 45 |
| 369407 | 868 | GTTTCCAAATCTGGATCCTC | 66 |
| 369408 | 959 | GAATAAATGTCCACCAGGCT | 35 |
| 369409 | 968 | TGTAGCTGGGAATAAATGTC | 69 |
| 369410 | 1121 | TGGAACTTGGTCTGAGTCTT | 80 |
| 369411 | 1128 | TCCAGCCTGGAACTTGGTCT | 83 |
| 369412 | 1152 | CCTCAAGCCCAACAGGAATC | 56 |
| 369413 | 1238 | CCCTGAGGCACACTCAGCTC | 74 |
| 369414 | 1243 | CAGGACCCTGAGGCACACTC | 72 |
| 369415 | 1250 | CCAGCCCCAGGACCCTGAGG | 75 |
| 369416 | 1286 | ACAGTGTTGTTGATGATTTC | 69 |
| 369417 | 1295 | TCCAAGGGCACAGTGTTGTT | 87 |
| 369418 | 1318 | AGCAGTTCCCAGGAATGCTG | 77 |
| 369419 | 1323 | AGAGCAGCAGTTCCCAGGAA | 68 |
| 369420 | 1328 | AGGGCAGAGCAGCAGTTCCC | 65 |
| 369421 | 1338 | GTTCTTGAACAGGGCAGAGC | 62 |
| 369422 | 1348 | TGAGAAGCAGGTTCTTGAAC | 55 |
| 369423 | 1353 | CTTCTTGAGAAGCAGGTTCT | 62 |
| 369424 | 1360 | GCTTGATCTTCTTGAGAAGC | 68 |
| 369425 | 1392 | TGTGACAGACTCAGTGCCCT | 84 |
| 369426 | 1424 | CTGGCAGAGAAGAGCACAGC | 63 |
| 369427 | 1429 | TGAAGCTGGCAGAGAAGAGC | 59 |
| 369428 | 1439 | GGGCCAAGTGTGAAGCTGGC | 73 |
| 369429 | 1471 | ACAGGGCCTGGAGCTGGATG | 40 |
| 369430 | 1477 | GCAGAGACAGGGCCTGGAGC | 59 |
| 369431 | 1585 | GCTCAGCCACCACAAAGGGC | 73 |
| 369432 | 1620 | GTTCAGAGTTTCACACATCT | 81 |
| 369433 | 1641 | CACCTCAGCCATGAACTTCA | 29 |
| 369434 | 1646 | GTCCCCACCTCAGCCATGAA | 35 |
| 369435 | 1651 | GGTTGGTCCCCACCTCAGCC | 83 |
| 369436 | 1672 | AGTGCTCTGGGAGCAGCCCC | 76 |
| 369437 | 1677 | GAGGAAGTGCTCTGGGAGCA | 67 |
| 369438 | 1686 | GGCCAGGAAGAGGAAGTGCT | 55 |
| 369439 | 1694 | ATCTTCTGGGCCAGGAAGAG | 11 |
| 369440 | 1699 | TGAAGATCTTCTGGGCCAGG | 47 |
| 369441 | 1704 | GTCATTGAAGATCTTCTGGG | 30 |
| 369442 | 1708 | TGTTGTCATTGAAGATCTTC | 65 |

**Table 4**

| **Inhibition of human STAT 6 mRNA levels by chimeric phosphorothioate oligonucleotides having 2'-MOE wings and a deoxy gap** | | | |
|---|---|---|---|
| **ISIS #** | **TARGET SITE** | **SEQUENCE (5' to 3')** | **% INHIB** |
| 342160 | 1821 | GTCCAGGACACCATCAAACC | 61 |
| 369452 | 1800 | CTGCCAAAAGGTGAAGCCAC | 64 |
| 369453 | 1816 | GGACACCATCAAACCACTGC | 72 |
| 369454 | 2004 | TGGAGAGCCATCCTGGCCCC | 53 |
| 369455 | 2024 | GGCTGGATGTTCTCTATCTG | 68 |
| 369456 | 2029 | AGAATGGCTGGATGTTCTCT | 63 |
| 369457 | 2034 | GGCAGAGAATGGCTGGATGT | 86 |
| 369458 | 2044 | ACAGGTCTTTGGCAGAGAAT | 70 |
| 369459 | 2050 | GAATGGACAGGTCTTTGGCA | 64 |
| 369460 | 2091 | TTTGAGCTGAGCAAGATCCC | 64 |
| 369461 | 2121 | CTCATCCTTGGGCTTCTTGG | 70 |
| 369462 | 2128 | GGAAAGCCTCATCCTTGGGC | 72 |
| 369463 | 2149 | GTTCAGGCTTGTAGTGGCTC | 58 |
| 369464 | 2175 | ATAACCCCTGCCATCCTTAC | 37 |
| 369465 | 2180 | GGGACATAACCCCTGCCATC | 79 |
| 369466 | 2190 | GATGGTAGCTGGGACATAAC | 57 |
| 369467 | 2199 | GGTCATCTTGATGGTAGCTG | 54 |
| 369468 | 2245 | TAGGCATCTGGAGCTCTGGG | 55 |
| 369469 | 2250 | CATGGTAGGCATCTGGAGCT | 62 |
| 369470 | 2263 | CATAAGAAGGCACCATGGTA | 20 |
| 369471 | 2270 | CCAAGGTCATAAGAAGGCAC | 76 |
| 369472 | 2278 | GGGCCATTCCAAGGTCATAA | 57 |
| 369473 | 2296 | TGCTCATGGAGGAATCAGGG | 33 |
| 369474 | 2301 | CTGCATGCTCATGGAGGAAT | 36 |
| 369475 | 2311 | CTGGGCCAAGCTGCATGCTC | 38 |
| 369476 | 2316 | CATATCTGGGCCAAGCTGCA | 28 |
| 369477 | 2321 | GGCACCATATCTGGGCCAAG | 53 |
| 369478 | 2341 | AGTGTGGTGGGTACACCTGG | 62 |
| 369479 | 2429 | GGCATCTGCAGGTGAGGCTC | 73 |
| 369480 | 2454 | CAGGCTCATCTGGCCCAGGC | 71 |
| 369481 | 2522 | GGGCTGGACACAGCATGCTC | 64 |
| 369482 | 2527 | GGTCAGGGCTGGACACAGCA | 74 |
| 369483 | 2547 | CACATCTGAGCAGAGCAGGG | 68 |
| 369484 | 2557 | CCACCATGGTCACATCTGAG | 83 |
| 369485 | 2563 | TGTCTTCCACCATGGTCACA | 67 |
| 369486 | 2584 | TCACTGGCTGGCTCAGGCAG | 67 |
| 369487 | 2613 | ACCAATCCAAGTGCCCTGAG | 73 |
| 369488 | 2618 | TCTTCACCAATCCAAGTGCC | 53 |
| 369489 | 2623 | ATATGTCTTCACCAATCCAA | 68 |
| 369490 | 2633 | AGAGGAGGGAATATGTCTTC | 52 |
| 369491 | 2639 | GGCAGCAGAGGAGGGAATAT | 47 |
| 369492 | 2669 | AGAAGCTTAGTGAGGTCCTG | 66 |
| 369493 | 2748 | AGATTGCCCATAGTGGGAGG | 50 |
| 369494 | 2754 | GATCCCAGATTGCCCATAGT | 62 |
| 369495 | 2759 | ATTGAGATCCCAGATTGCCC | 69 |
| 369496 | 2764 | GGGACATTGAGATCCCAGAT | 65 |
| 369497 | 2774 | AGGTCCATGTGGGACATTGA | 43 |
| 369498 | 2781 | GGCCCTTAGGTCCATGTGGG | 56 |
| 369499 | 2786 | GGGTTGGCCCTTAGGTCCAT | 72 |
| 369500 | 2864 | AAGTGTCCAGAGCAGGTCTG | 87 |
| 369501 | 2894 | TCCCCATCTGCTGCTTGGCA | 74 |
| 369502 | 3034 | ACTTCCCTTCCAGTCAGTGC | 76 |
| 369503 | 3040 | GCCTGAACTTCCCTTCCAGT | 90 |
| 369504 | 3267 | AGACCCAATATCCTCTATCC | 56 |
| 369505 | 3272 | GGCTGAGACCCAATATCCTC | 86 |
| 369506 | 3303 | GGGTCCCTTGAGCTGCTTCC | 42 |
| 369507 | 3340 | TAACCACATGTCCAGACCCC | 67 |
| 369508 | 3440 | TACTTTTGCATAGTCTCATA | 83 |
| 369509 | 3447 | GCCCTTGTACTTTTGCATAG | 71 |
| 369510 | 3540 | TGTTCTATGTGGTCATGCAA | 82 |
| 369511 | 3545 | ACACATGTTCTATGTGGTCA | 84 |
| 369513 | 3627 | GAGGCCAGCACACTTGCTGC | 53 |
| 369515 | 3642 | TTAGCATATGTCAGAGAGGC | 86 |
| 369516 | 3684 | CACTTGGGCACAGTCAGACT | 78 |
| 369518 | 3689 | GGACCCACTTGGGCACAGTC | 84 |
| 369520 | 3694 | CACTTGGACCCACTTGGGCA | 75 |
| 369522 | 3704 | ATGTCACAGCCACTTGGACC | 71 |
| 369523 | 3761 | GACCCAGACTCTCACCCTGG | 82 |
| 369524 | 3768 | AGGCCTGGACCCAGACTCTC | 52 |
| 369525 | 3793 | TCATACACTGGAGGGCCACA | 62 |
| 369526 | 3899 | GCTTAGGATCTATGACCCCT | 83 |

The screen identified active target segments within the human STAT 6 mRNA sequence. Each active target segment was targeted by multiple, active antisense oligonucleotides. These regions include nucleotides 615-658; 1121-1171; 1318-14112929-2967; 2522-2582; 3540-3564; and 3761-3787 of SEQ ID NO: 1. All of the oligonucleotides tested within each of these regions inhibited expression of human STAT 6 RNA at least 50%, and over half of the oligonucleotides tested inhibited expression at least 65%. The screen also identified inactive target segments, regions to which multiple inactive antisense oligonucleotides were targeted. These regions include nucleotides 1641-1665 and 2296-2335 of SEQ ID NO: 1. All of the oligonucleotides tested inhibited expression of human STAT 6 RNA 38% or less. Identification of these regions allows for the design of antisense oligonucleotides that modulate the expression of STAT 6.

Oligonucleotides targeted to the following sites on SEQ ID NO: 1 inhibit expression of human STAT 6 RNA at least about 65% under the conditions described for the respective target sites above: 20-39, 27-46, 29-48, 145-164, 154-173; 185-204; 252-271; 258-277, 263-282, 264-283, 284-303, 378-397, 383-402, 431-450; 439-458,497-516,498-517, 523-542, 525-544, 561-580, 615-634, 620-639, 630-649, 641-660, 968-987, 750-769, 760-779, 770-789, 775-794, 824-843, 835-854, 868-887, 871-890, 900-919, 1023-1042, 1121-1140, 1128-1147, 1160-1179,1191-1210, 1238-1257, 1243-1262, 1250-1269, 1266-1285, 1277-1296, 1286-1305, 1295-1314, 1311-1330, 1318-1337, 1323-1342, 1328-1347, 1343-1362, 1360-1379, 1392-1411, 1439-1458, 1558-1577, 1585-1604, 1607-1626, 1620-1639, 1651-1670, 1672-1691, 1677-1696, 1708-1727, 1721-1740, 1777-1796, 1816-1835, 1826-1845, 1831-1850, 1849-1868, 1868-1887, 1903-1922, 1911-1930, 1925-1944, 2011-2030, 2022-2041, 2024-2043, 2034-2053, 2044-2063, 2050-2069, 2053-2072, 2057-2076, 2063-2082, 2078-2097, 2121-2140, 2128-2147, 2180-2199, 2270-2289, 2271-2290, 2427-2446, 2429-2448, 2454-2473, 2527-2546, 2547-2566, 2557-2576, 2563-2582, 2584-2603, 2585-2604, 2589-2608, 2612-2631, 2613-2632, 2623-2642, 2656-2675, 2669-2688, 2759-2778, 2764-2783, 2782-2801, 2786-2805, 2864-2883, 2894-2913, 3034-3053, 3040-3059, 3050-3069, 3106-3125, 3125-3144, 3177-3196, 3222-3241, 3272-3291, 3340-3359, 3440-3459, 3447-3466, 3523-3542, 3531-3550, 3540-3559, 3545-3564, 3577-3596, 3621-3640,3642-3661, 3684-3703, 3689-3708, 3694-3713, 3704-3723, 3761-3780, 3889-3918. An active target segment can be bracketed by any of the two segments listed above, provided that the requirements for activity of the intervening oligonucleotides is met. Using the list and the tables above, a subset of oligonucleotides with activity of at least about 70%, at least about 75%, at least about 80%, at least about 85%, and at least about 90% can be readily identified. Such analyses are well within the ability of those skilled in the art.

### Example 4: Antisense inhibition of murine STAT 6 expression by antisense compounds

A series of antisense antisense compounds was designed to target different regions of mouse STAT 6 RNA, using published sequences cited in Table 1. In the instant screen, b.END cells were treated with 45nM of oligonucleotide using LIPOFECTIN™. Inhibition of mRNA target expression was determined using the RT-PCR method detailed above. From this screen, two of the oligonucleotides found to be active were selected for further analysis, ISIS 195428 (5'-CCACAGAGACATGATCTGGG-3', SEQ ID NO: 2) and ISIS 342133 (5'-CCGACCAGGAACTCCCAGGG-3', SEQ ID NO: 3).

Both of the STAT 6 specific ASOs gave a dose dependent reduction in the target RNA. No significant change in RNA levels were observed with a control, non-specific ASO. This demonstrates that the STAT 6 ASOs are working via a target specific mechanism.

### Example 5: Mouse models of allergic inflammation

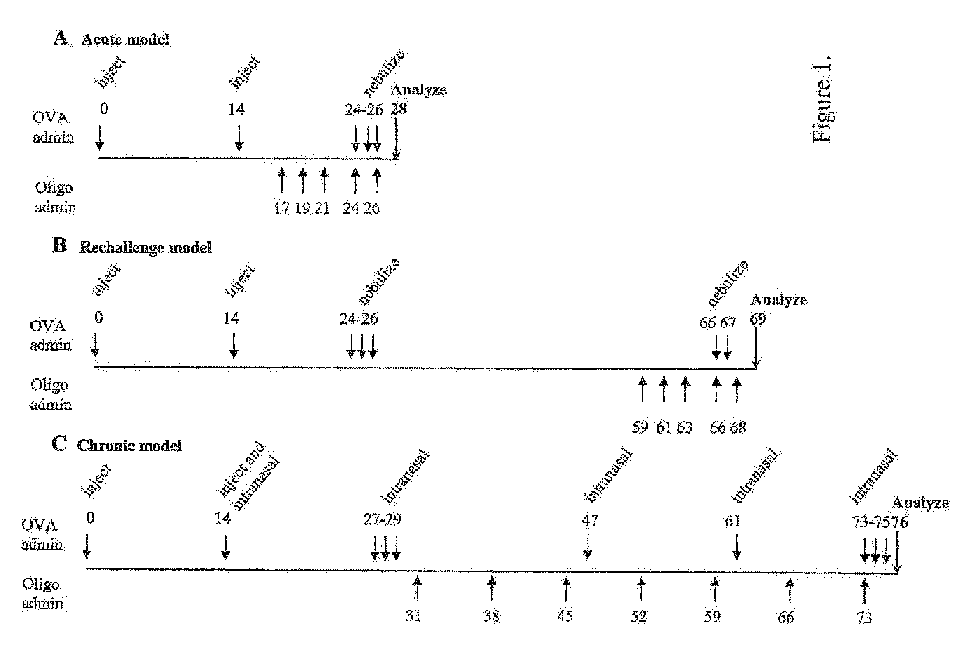

Asthma is a complex disease with variations on disease severity and duration. In view of this, multiple animal models have been designed to reflect various aspects of the disease (see Figure 1). It is understood that the models have some flexibility in regard to days of sensitization and treatment, and that the timelines provided reflect the experimental methods used herein. There are several important features common to human asthma and the mouse model of allergic inflammation. One of these is pulmonary inflammation, in which production of Th2 cytokines, e.g., IL 4, IL 5, IL 9, and IL 13 is dominant. Another is goblet cell metaplasia with increased mucus production. Lastly, airway hyperresponsiveness (AHR) occurs, resulting in increased sensitivity to cholinergic receptor agonists such as acetylcholine or methacholine.

### Ovalbumin induced allergic inflammation-acute model

The acute model of induced allergic inflammation is a prophylaxis treatment paradigm. Animals are sensitized to allergen by systemic administration (i.e., intraperitoneal injection), and treated with the therapeutic agent prior to administration of the pulmonary allergen challenge (see Figure 1A). In this model, there is essentially no pulmonary inflammation prior to administration of the therapeutic agent.

Balb/c mice (Charles River Laboratory, Taconic Farms, NY) were maintained in micro-isolator cages housed in a specific pathogen free (SPF) facility. The sentinel cages within the animal colony surveyed negative for viral antibodies and the presence of known mouse pathogens. Mice were sensitized and challenged with aerosolized chicken OVA. Briefly, 20 ug of alum precipitated OVA was injected intraperitoneally on days 0 and 14. On days 24, 25 and 26, the animals were exposed for 20 minutes to 1% OVA (in saline) by ultrasonic nebulization using a Lovelace nebulizer (Model 01-100). On days 17, 19, 21, 24, and 26 animals were dosed intratracheally with 0.01 ug/kg, 0.1 ug/kg, 10ug/kg or 100 ug/kg of ISIS 195428 or ISIS 342133 as well as a mismatch control oligonucleotide and/or vehicle control (0.9% normal saline). Analysis was performed on day 28.

### Effect of pulmonary administration of ASOs targeted to STAT 6 on airway hyperreponsiveness inresponse to methacholine

Airway responsiveness was assessed by inducing airflow obstruction with a methacholine aerosol using a noninvasive method. This method used unrestrained conscious mice that are placed into a test chamber of a plethsmograph (Buxco Electronics, Inc. Troy, NY). Pressure difference between this chamber and a reference chamber were used extrapolate minute volume, breathing frequency and enhanced pause (Penh). Penh is a dimensionless parameter that is a function of total pulmonary airflow in mice (i.e. the sum of the airflow in the upper and lower respiratory tracts) during the respiratory cycle of the animal. A lower Penh is indicative of greater the airflow. This parameter is known to closely correlate with lung resistance as measured by traditional, invasive techniques using ventilated animals (see e.g., Hamelmann et al., 1997).

ISIS195428 or ISIS 342133, but not the vehicle or mismatch oligonucleotide control, caused a significant (p < 0.05 v. control) suppression in methacholine induced AHR at all doses in sensitized mice as measured by whole body plethysmography.

### Effect of pulmonary administration of ASOs targeted to STAT 6 on inflammatory cell infiltration

The effect of ISIS195428 and ISIS 342133 on inflammatory cell profiles, particularly eosinophils, was analyzed. Cell differentials were performed on bronchial alveolar lavage (BAL) fluid collected from lungs of the treated mice after injection of a lethal dose of ketamine. Treatment with ISIS195428 and ISIS 342133, but not the vehicle or mismatch oligonucleotide control, resulted in a trend towards a decrease in BAL eosinophil (eos) infiltration. These results suggest that an oligonucleotide targeted to STAT 6 decreased pulmonary inflammation by decreasing eosinophil infiltration.

These data demonstrate that STAT 6 targeted antisense oligonucleotide approach is efficacious in decreasing pulmonary inflammation and airway hyperresponsiveness in a prophylaxis model, and that STAT 6 is an appropriate target for the prevention, amelioration, and/or treatment of AHR and pulmonary inflammation, and diseases associated therewith.

### Mouse model of allergic inflammation- rechallenge model

The rechallenge model of induced allergic inflammation allows testing of a pharmacologic approach in mice that have been previously sensitized and then exposed to an aeroallergen. During the first set of local allergen challenges, the mice develop allergen-specific memory T lymphocytes. Subsequent exposure to a second set of inhaled allergen challenges produces an enhanced inflammatory response in the lung, as demonstrated by increased levels of Th2 cytokines in lavage fluid. The rechallenge model of allergic inflammation includes a second series of aerosolized administration of OVA on days 59 and 60 in addition to the two IP OVA administrations on days 0 and 14 and the nebulized OVA administration of days 24, 25 and 26 of the acute model (see Figure 1B). Using this model, oligonucleotide treatment occurs after the first set of local allergen challenges. This also allows for the evaluation of the target's role in a recall response, as opposed to an initial immune response.

In the rechallenge model, mice were treated with ISIS 195428; and a mismatch control oligonucleotide and/or a vehicle control (i.e., 0.9% normal saline) on days 59, 61, 63, 66, and 68 delivered by nose only inhalation. In one experiment, oligonucleotides were dosed at 10, 100, and 500 ug/kg. In another experiment, oligonucleotides were dosed at 0.1, 1, and 10 ug/kg. A Lovelace nebulizer (Model 01-100) was used to deliver the oligonucleotide into an air flow rate of 1.0 liter per minute feeding into a total flow rate of 10 liters per minute. The exposure chamber was equilibrated with an oligonucleotide aerosol solution for 5 minutes before mice were placed in a restraint tubes attached to the chamber. Restrained mice were treated for a total of 10 minutes. The study endpoints can include many of those used in the acute model: Penh response (i.e., AHR reduction), inflammatory cells in BAL, mucus accumulation, cytokine production, and lung histology. STAT 6 RNA and protein level reductions in pulmonary structural and inflammatory cells can also be evaluated.

A significant (p < 0.05 v. control) reduction in Penh was observed at the 0.1, 10, 100, and 500 ug/kg doses of ISIS 195428. A significant (p < 0.05 v. control) reduction in eosinophils in BAL was observed at all five doses of ISIS 195428.

These data demonstrate that STAT 6 targeted antisense oligonucleotide approach is efficacious in decreasing pulmonary inflammation and airway hyperresponsiveness, and that STAT 6 is an appropriate target for the prevention, amelioration, and/or treatment of AHR, pulmonary inflammation, and diseases associated therewith.

### Mouse model of allergic inflammation- chronic model

The chronic model of induced allergic inflammation uses a therapeutic treatment regimen, with ASO treatment initiated after the establishment local pulmonary inflammation. The chronic model recapitulates some of the histological features of severe asthma in humans, including collagen deposition and lung tissue remodeling. The chronic OVA model produces a more severe disease than that observed in the acute or rechallenged model.

This model includes intranasal OVA administration on days 14, 27-29, 47, 61, and 73-75, at a higher dose (500 ug) than in the acute and chronic models, in addition to the two OVA IP administrations on days 0 and 14 (see Figure 1C). ISIS 195428 and a vehicle control were administered by nose-only aerosol at doses of 5 and 500 ug/kg on days 31, 38, 45, 52, 59, 66, and 73. Analysis of endpoints was performed on day 76. BAL inflammatory cells were also measured on day 62. Intranasal administration of the allergen results in a higher dose of the allergen delivered to the lungs relative to delivery by nebulizer. The increased number of allergen challenges produces more severe inflammatory events, resulting in increased lung damage and pathology more reflective of clinical asthma than other models, in the absence of therapeutic interventions. Endpoints tested can include those in the acute and rechallenge model, such as Penh (AHR), BAL inflammatory cells and cytokines, lung histology, and mucus accumulation. A "lung inflammation score" was also determined in this experiment. The score is a combination of a number of factors including PAS positive airways, inflammatory cell infiltrates, goblet cell hyperplasia, and other indicators of inflammation. This model also allows for the analysis of endpoints typically associated with chronic diseases, such as asthma and COPD, including subepithelial fibrosis, collagen deposition, enhanced goblet cell metaplasia, and smooth muscle cell hyperplasia.

A significant (p < 0.05 v. control) reduction in Penh was observed at both the 5 and 500 ug/kg doses of ISIS 195428. A significant (p < 0.05 v. control) reduction in eosinophils and neutrophils in BAL was observed to the 500 ug/kg dose on day 76. A significant (p < 0.05 v. control) reduction in eosinophils was observed with both the 5 ug/kg and 500 ug/kg doses on day 62. A significant (p < 0.05 v. control) decrease in lung inflammation score was also observed in response to both doses of ISIS 195423.

These data demonstrate that STAT 6 targeted antisense oligonucleotide approach is efficacious in decreasing pulmonary inflammation and airway hyperresponsiveness, and that STAT 6 is an appropriate target for the prevention, amelioration, and/or treatment of AHR and pulmonary inflammation, and diseases associated therewith.

### Example 6: Mouse model of allergic inflammation, analysis for nasal rhinitis endpoints

Mouse models of allergen - induced acute and chronic nasal inflammation similar to the allergic inflammation models above have been used to study allergic rhinitis in mice (Hussain et al., Larangyoscope. 112: 1819-1826. 2002; Iwasaki et al., J. Allergy Clin Immunol. 112: 134-140. 2003; Malm-Erjefaelt et al., Am J Respir Cell Mol Biol. 24:352-352.2001; McCusker et al., J Allergy Clin Immunol., 110: 891-898; Saito et el., Immunology. 104:226-234. 2001). In all of the models, the mice are sensitized to OVA by injection, as above, followed by intranasal OVA instillation.

The most substantial difference in the models is in the endpoints analyzed. Endpoints include, but are not limited to, the amount of sneezing and nasal scratching immediately after administration of allergen challenge (i.e. intranasal OVA), and nasal histology including mucus and eosinophil counts and measurements of cytokines or other inflammatory products in nasal lavage fluid or nasal tissues. Methods for performing such analyses are detailed in the references cited which are incorporated herein by reference.

### Example 7: Rodent model of smoking induced pulmonary disease

Smoking is known to cause lung irritation and inflammation which can result in a number of diseases in humans including, but not limited to, emphysema and COPD. A number of smoking animal models are well known to those skilled in the art including those utilizing mice (Churg et al., 2002. Am. J. Respir. Cell. Mol. Biol. 27:368-347; Churg et al., 2004. Am. J. Respir. Crit. Care Med. 170:492-498, both incorporated herein by reference), rats (e.g., Sekhon et al., 1994. Am. J. Physiol. 267:L557-L563, incorporated herein by reference), and guinea pigs (Selman et al., 1996. Am J. Physiol. 271:L734-L739, incorporated herein by reference). Animals are exposed to whole smoke using a smoking apparatus (e.g., Sekhon et al., 1994. Am. J. Physiol. 267:L557-L563) well known to those skilled in the art.

Changes in lung physiology are correlated with dose and time of exposure. In short term studies, cell proliferation and inflammation were observed. In one study, exposure of rats to 7 cigarettes for 1, 2, or 7 days resulted in proliferation of pulmonary artery walls at the level of the membranous bronchioles (MB), respiratory bronchioles (RB), and alveolar ducts (AD). Endothelial cell proliferation was only present in vessels associated with AD. In a separate study (Churg et al., 2002. Am. J. Respir. Cell. Mol. Biol. 27:368-347), mice exposed to whole smoke from four cigarettes were shown to have an increase in neutrophils, desmosine (an indicator of elastin breakdown), and hydroxyproline (an indicator of collagen breakdown) after only 24 hours. In a long term study, an emphysema-like state was induced (Churg et al., 2004. Am. J. Respir. Crit. Care Med. 170:492-498). Mice exposed to whole smoke from four cigarettes using a standard smoking apparatus, for five days per week for six months were found to have an increase in neutrophils and macrophages in BALF as compared to control mice. Whole lung matrix metalloproteinases (MMP)-2, -9, -12, and -13, and matrix type-1 (MT-1) proteins were increased. An increase in matrix breakdown products was also observed in BALF. These markers correlate with tissue destruction and are observed in human lungs with emphysema.

These models can be used to determine the efficacy of therapeutic interventions for the prevention, amelioration, and/or treatment of the damage and disease caused by cigarette smoke and/or other insults. Administration of oligonucleotide can be performed prior to, concurrent with, and/or after exposure to smoke to provide a prophylactic or therapeutic model. Both ISIS195428 and ISIS 342133 are 100% complimentary to both mouse and rat STAT 6; therefore, they can be used in both mouse and rat studies. Dose ranges are determined by the time of oligonucleotide administration relative to smoke inhalation, with lower doses (e.g., 1-100 ug/kg) required for prevention of lung damage. Higher doses (e.g., 100-1000 ug/kg) are required for treatment after, or alternating with, smoke exposure. Positive control (e.g., smoke exposure, no oligonucleotide administration) and negative control (e.g., no smoke exposure, with or without oligonucleotide treatment) animals are also analyzed.

Endpoints for analysis include those discussed in the asthma models above. Functional endpoints include AHR, resistance and compliance. Morphological changes include BAL cell, cytokine levels, histological determinations of alveolar destruction (i.e., increase in alveolar space) and airway mucus accumulation, as well as tissue markers of disease including collagen and elastin. The emphysematous changes specific to this model discussed in this example can also be analyzed to determine the effect of the antisense oligonucleotide.

### Example 8: Mouse model of elastase induced emphysema

Elastase is an essential mediator in lung damage and inflammation release by neutrophils recruited following smoke-induced damage. A rat model of emphysema has been developed to analyze the process of elastase mediated lung damage, and possible therapeutic interventions to prevent, ameliorate, and/or treat the pathologies associated with such damage and resulting disease (Kuraki et al., 2002. Am J Respir Crit Care Med., 166:496-500, incorporated herein by reference). Intratracheal application of elastase induced emphysematous changes in all lobes of the lung including severe lung hemorrhage as demonstrated by increased hemoglobin in BALF; neutrophil accumulation in BALF; inhibition of hyperinflation and degradation of elastic recoil. Histopathological changes included elastase-induced airspace enlargement and breakdown of alveoli. These changes are similar to those observed in human emphysema.

In the model, rats are treated with human sputum elastase (SE563, Elastin Products, Owensville, MO) without further purification. Rats are treated with a sufficient dose of elastase, about 200 to 400 units, by intratracheal administration using a microsprayer. Alternatively, intratracheal administration can be performed as described above in the mouse models. After sufficient time to allow for damage to occur, about eight weeks, functional and morphological changes are analyzed. A similar model can be performed using mice with a lowered dose of elastase relative to weight and/or lung area (e.g., 0.05 U of porcine pancreatic elastase/g body weight).

Administration of oligonucleotide can be performed prior to, concurrent with, and/or after administration of elastase to provide a prophylactic or therapeutic model. Both ISIS 195428 and ISIS 342133 are 100% complimentary to both mouse and rat STAT 6. Dose ranges are determined by the time of oligonucleotide administration relative to elastase administration with lower doses (e.g., 1-100 ug/kg) required for prevention of lung damage. Higher doses (e.g., 100-1000 ug/kg) are required for treatment after, or alternating with, elastase administration. Positive control (e.g., elastase treatment, no oligonucleotide administration) and negative control (e.g., no elastase, with or without oligonucleotide treatment) animals are also analyzed.

Endpoints for analysis include those discussed in the asthma models above. Functional endpoints include AHR, resistance and compliance. Morphological changes include BAL cell, cytokine levels, and mucus accumulation. The emphysematous changes specific to this model discussed in this example can also be analyzed to determine the effect of the antisense oligonucleotide.

### Embodiments of the Invention:

1. An antisense compound of 15 to 35 nucleobases targeted to a nucleic acid molecule encoding human STAT 6 (SEQ ID NO: 1), wherein the compound is targeted to at least a 15-nucleobase portion of nucleotides 615-658; 1121-1171; 1318-1411; 2929-2967; 2522-2582; 3540-3564; or 3761-3787 of SEQ ID NO: 1.
2. The compound of embodiment 1 , wherein the compound is at least about 80% identical to a 20 nucleobase portion 100% complementary to nucleotides 615-658; 1121-1171; 1318-1411; 2929-2967; 2522-2582; 3540-3564; or 3761-3787 of SEQ ID NO: 1.
3. The compound of embodiment 1 or 2, wherein the compound is a single stranded compound.
4. The compound of embodiment 1, 2 or 3, wherein the compound is an antisense oligonucleotide.
5. The compound of embodiment 4 having at least one modified internucleoside linkage, sugar moiety, or nucleobase.
6. The compound of embodiment 4 or 5 comprising a chimeric oligonucleotide.
7. The compound of embodiment 4 or 5 wherein the modified internucleoside linkage comprises a phosphorothioate linkage.
8. The compound of embodiment 4 or 5 wherein the modified sugar moiety comprises a 2'-MOE modification.
9. The compound of embodiments 4 or 5 wherein the modified nucleobase comprises 5- methylcytosine.
10. A pharmaceutical composition comprising a compound of any of embodiments 1 to 9 and a pharmaceutically acceptable penetration enhancer, carrier, or diluent.
11. A use of the composition of any of embodiments 1 to 10 for formulation of a medicament for prevention, amelioration, or treatment of pulmonary inflammation or airway hyperresponsiveness comprising administration of the compound of embodiment 1 to an individual in need of such intervention
12. The use of embodiment 11 wherein administration comprises topical administration to a respiratory tract of an animal.
13. The use of embodiment 11 wherein administration is comprises pulmonary administration.
14. The use of embodiment 11 wherein administration comprises aerosol administration.

## Claims

1. An antisense compound 12 to 35 nucleobases in length targeted to a nucleic acid molecule encoding human STAT6, wherein said compound is at least 80% complementary to at least a 15 nucleobase portion of SEQ NO:1.

2. The compound of claim 1 comprising a chimeric oligonucleotide.

3. The compound of claim 1, wherein the compound is a single-stranded compound.

4. The compound of claim 3, wherein the compound is an antisense oligonucleotide.

5. The compound of any preceding claim comprising at least one modified internucleoside linkage, sugar moiety, or nucleobase.

6. The compound of claim 5, wherein the modified internucleoside linkage comprises a phosphorothioate internucleoside linkage.

7. The compound of claim 5, wherein the modified sugar moiety comprises a 2'-MOE modification.

8. The compound of claim 5, wherein the modified sugar moiety is a bicyclic sugar, wherein optionally the bicyclic sugar is LNA or ENA.

9. The compound of claim 5, wherein the modified nucleobase comprises 5-methylcytosine.

10. The compound of any preceding claim, wherein the compound is 15-35 nucleobases in length.

11. A pharmaceutical composition comprising a compound of any preceding claim and a pharmaceutically acceptable penetration enhancer, carrier or diluent.

12. A compound of any of claims 1-10, for use in therapy for a disease or condition associated with STAT6.

13. A compound of any of claims 1-10, for use in the prevention, amelioration or treatment of pulmonary inflammation or hyper-responsiveness.

14. A compound of any of claims 1-10, for use in the treatment of asthma or allergic rhinitis.

15. The compound for use according to any of claims 12-14, wherein said compound is administered by:
a) topical administration to a respiratory tract of an animal;
b) pulmonary administration; or
c) aerosol administration.
